# FASCICULE DE BREVET EUROPEEN

(11) **EP 0 703 907 B1**
(45) Date de publication et mention de la délivrance du brevet: **13.08.1997**
(21) Numéro de dépôt: 94919710.7
(22) Date de dépôt: 15.06.1994
(51) Int. Cl.: C07D 263/04, C07D 305/14

(54) **PROCEDE DE PREPARATION D'UN ACIDE OXAZOLIDINECARBOXYLIQUE UTILE POUR PREPARER DES TAXOIDES THERAPEUTIQUEMENT ACTIFS**
VERFAHREN ZUR HERSTELLUNG EINER OXAZOLIDINCARBON SÄURE NÜTZLICH FÜR DIE HERSTELLUNG VON THERAPEUTISCHEN TAXOIDEN
METHOD FOR PREPARING AN OXAZOLIDINECARBOXYLIC ACID USEFUL FOR PREPARING THERAPEUTICALLY ACTIVE TAXOIDS

(30) Priorité: 16.06.1993 FR 9307240
(43) Date de publication de la demande: 03.04.1996
(73) Titulaire: RHONE-POULENC RORER S.A., 92160 Antony (FR)
(72) Inventeur: BOURZAT, Jean-Dominique, F-94300 Vincennes (FR); COMMERçON, Alain, F-94400 Vitry-sur-Seine (FR)
(74) Mandataire: Pilard, Jacques
(86) Numéro de dépôt international: FR9400713
(87) Numéro de publication internationale: WO9429284

(56) Documents cités:
- WO-A-92/09589

## Description

La présente invention concerne un procédé de préparation d'un acide oxazolidinecarboxylique ou de ses dérivés de formule générale : 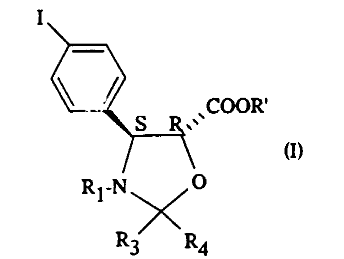 à partir d'un acide ou de ses dérivés de formule générale :

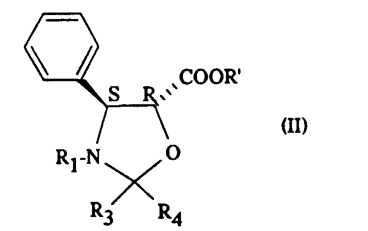

Dans les formules (I) et (II),
R' représente un atome d'hydrogène ou un atome de métal alcalin ou alcalino-terreux ou un radical alcoyle contenant 1 à 4 atomes de carbone éventuellement substitué par un radical phényle,
R₁ représente un radical benzoyle ou un radical R₂-O-CO- dans lequel R₂ représente :
   - un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone, cycloalcoyle contenant 4 à 6 atomes de carbone, alcényle contenant 4 à 6 atomes de carbone, cyano, carboxy ou alcoyloxycarbonyle dont la partie alcoyle contient 1 à 4 atomes de carbone,
   - ou un radical phényle éventuellement substitué par un ou plusieurs atomes ou radicaux choisis parmi les atomes d'halogène et les radicaux alcoyles contenant 1 à 4 atomes de carbone ou alcoyloxy contenant 1 à 4 atomes de carbone,
   - ou un radical hétérocyclyle azoté saturé ou non saturé contenant 5 ou 6 chaînons et éventuellement substitué par un ou plusieurs radicaux alcoyles contenant 1 à 4 atomes de carbone,
R₃ et R₄, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₃ représente un radical alcoxy contenant 1 à 4 atomes de carbone ou un radical trihalométhyle tel que trichlorométhyle ou un radical phényle substitué par un radical trihalométhyle tel que trichlorométhyle et R₄ représente un atome d'hydrogène, ou bien R₃ et R₄ forment ensemble avec l'atome de carbone auquel ils sont liés un cycle ayant 4 à 7 chaînons.

L'acide de formule générale (I) est particulièrement utile pour préparer les taxoïdes thérapeutiquement actifs de formule générale : 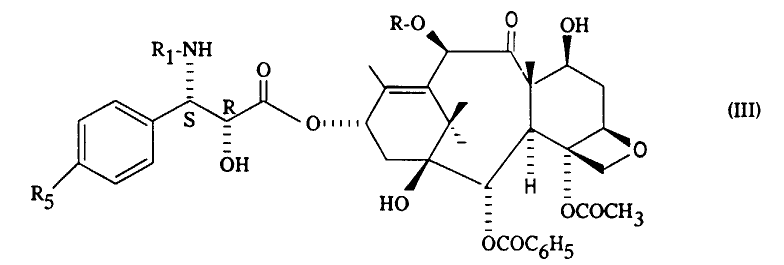 dans laquelle :
R représente un atome d'hydrogène ou le radical acétyle,
R₁ est défini comme précédemment, et
R₅ représente un atome d'iode ou un radical alcényle contenant 2 à 8 atomes de carbone éventuellement substitué par un radical phényle, alcynyle contenant 2 atomes de carbone, phényle, formyle, alcanoyle, aroyle, hydroxyméthyle, carboxy ou alcoxycarbonyle.

Selon la demande PCT WO 9209589, la préparation d'un produit de formule générale (III) nécessite la mise en oeuvre d'un acide oxazolidinecarboxylique de formule générale : 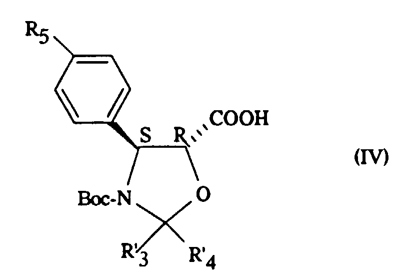 dans laquelle R₅ est défini comme précédemment, R'₃ et R'₄, identiques ou différents, représentent un radical alkyle contenant 1 à 4 atomes de carbone éventuellement substitué par un ou plusieurs radicaux aryles (phényle), ou aryle (phényle), ou bien R'₃ et R'₄ forment ensemble avec l'atome de carbone auquel ils sont liés un cycle ayant de 4 à 7 chaînons, et Boc représente le radical t.butoxycarbonyle, qui est obtenu à partir d'un aldéhyde de formule générale : 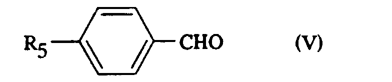 dans laquelle R₅ est défini comme précédemment, qui, selon les significations de R₅, n'est pas toujours facilement accessible.

Selon l'invention, le produit de formule générale (I) dans laquelle R' représente un radical alkyle éventuellement substitué par un radical phényle est obtenu par iodation selon les méthodes habituelles d'un produit de formule générale (II) dans laquelle R' représente un radical alkyle éventuellement substitué par un radical phényle.

Généralement, l'iodation peut être effectuée, ou bien au moyen d'iode en présence de bis-(trifluoroacétoxy)iodobenzène en opérant dans un solvant organique tel qu'un hydrocarbure aliphatique halogéné comme le dichlorométhane à une température comprise entre 0 et 50°C, ou bien par action de l'iode en présence de nitrate d'ammonium cérique dans l'acide acétique ou le méthanol, ou bien par action de l'iode en présence de trifluoroacétate d'argent, ou bien par action du N-iodosuccinimide en présence d'hydroxy toxyloxy iodobenzène (réactif de Koser) dans le méthanol, ou bien par action du dichloroiodure de benzyltriméthylammonium en présence de chlorure de zinc dans l'acide acétique.

Le produit de formule générale (I) pour lequel R' représente un atome d'hydrogène peut être obtenu par saponification d'un produit de formule générale (I) dans laquelle R' représente un radical alkyle éventuellement substitué par un radical phényle.

Le produit de formule générale (II) dans laquelle R' représente un radical alcoyle éventuellement substitué par un radical phényle, R₃ et R₄ sont définis comme précédemment, peut être obtenu par action d'un produit de formule générale : 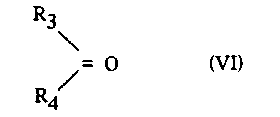 dans laquelle R₃ et R₄ sont définis comme précédemment sous forme d'un dialkylacétal ou d'un alkyléther d'énol, sur un ester de formule générale : 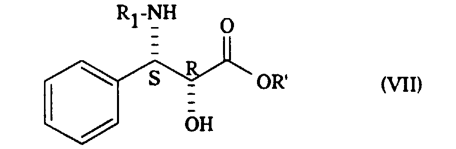 dans laquelle R₁ et R' sont définis comme précédemment qui peut être obtenu dans les conditions décrites dans la demande PCT WO 9209589.

Le produit de formule générale (I) peut être transformé en produit de formule générale (III) selon l'une des méthodes suivantes :
1) après remplacement de l'atome d'iode du produit de formule générale (I), dans laquelle R' représente un radical alcoyle éventuellement substitué par un radical phényle, par un radical alcényle contenant 2 à 8 atomes de carbone éventuellement substitué par un radical phényle, alcynyle contenant 2 atomes de carbone, phényle, formyle, alcanoyle, aroyle, hydroxyméthyle, carboxy ou alcoxycarbonyle selon les méthodes connues, et saponification, le produit obtenu de formule générale : 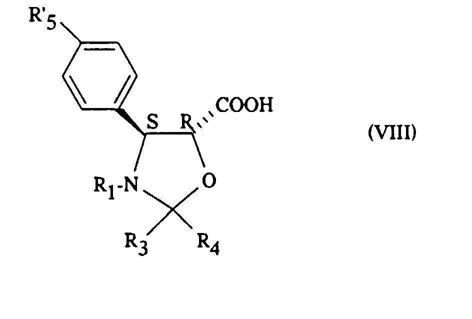 dans laquelle R₁, R₃ et R₄ sont définis comme précédemment, et R'₅ représente un radical alcényle éventuellement substitué par un radical phényle, alcynyle, phényle, alcanoyle, aroyle, formyle, hydroxyméthyle, carboxy ou alcoxycarbonyle, ou un dérivé de l'acide de formule générale (VIII), est condensé sur une baccatine III protégée de formule générale : 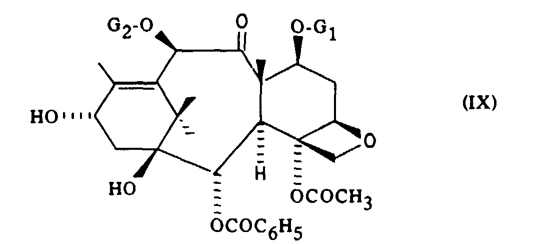 dans laquelle G₁ représente un groupement protecteur de la fonction hydroxy et G₂ représente un radical acétyle ou un groupement protecteur de la fonction hydroxy pour obtenir un produit de formule générale : 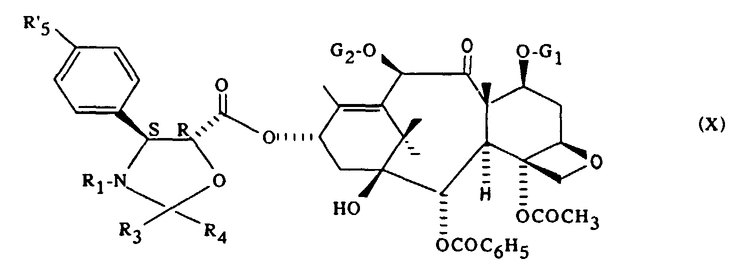 dans laquelle R₁, R₃, R₄, R'₅, G₁ et G₂ sont définis comme précédemment, dont les groupements protecteurs représentés par G₁ et G₂ sont remplacés par des atomes d'hydrogène en passant intermédiairement, selon les significations de R₃ et R₄, par un produit de formule générale : 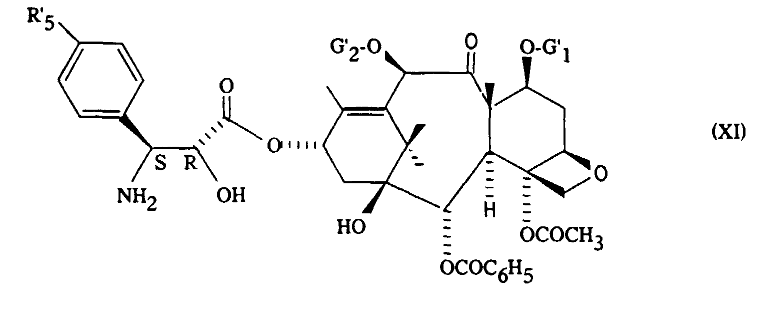 dans laquelle R'₅ est défini comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy, dont la fonction amine est acylée avant de remplacer les groupements protecteurs G'₁ et éventuellement G'₂ par des atomes d'hydrogène.
2) le produit de formule générale (I), dans laquelle R' représente un atome d'hydrogène, est condensé sur une baccatine III protégée de formule générale (IX) pour donner un produit de formule générale : 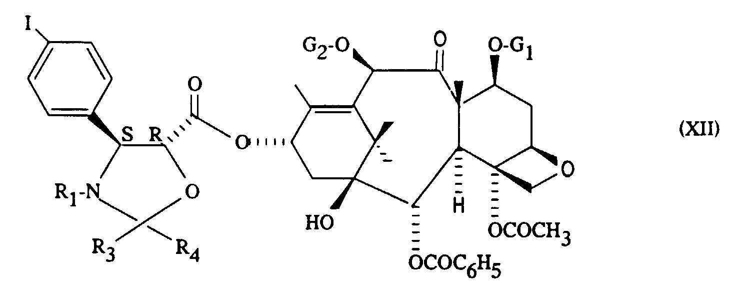 dans laquelle R₁, R₃, R₄, G₁ et G₂ sont définis comme précédemment, dont les groupements protecteurs représentés par G₁ et G₂ sont remplacés par des atomes d'hydrogène en passant intermédiairement, selon les significations de R₃ et R₄, par un produit de formule générale : 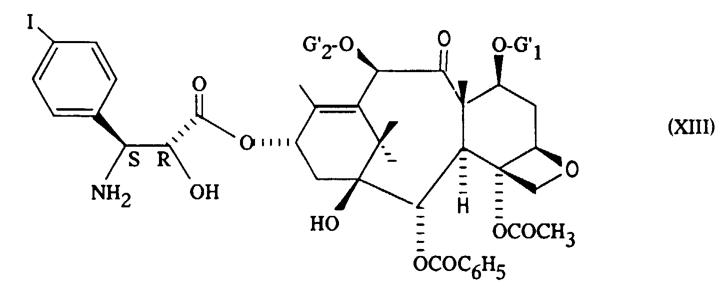 dans laquelle G'₁ et G'₂ sont définis comme précédemment, dont la fonction amine est acylée, avant de remplacer les groupements G'₁ et éventuellement G'₂ par des atomes d'hydrogène puis l'atome d'iode par un radical R'₅ tel que défini précédemment.
3) un produit de formule générale (III) peut être obtenu également à partir d'un produit de formule générale (III) préparé selon l'enchaînement décrit précédemment sous 1) par toute méthode permettant de transformer un substituant R'₅ en un autre substituant R'₅, par exemple, un produit de formule générale (III) dans laquelle R'₅ représente un radical vinyle peut être transformé, par ozonolyse, en un autre produit de formule générale (m) dans laquelle R'₅ représente un radical formyle.

Selon les significations de R'₅, le produit de formule générale (VIII), sous forme d'ester, peut être préparé selon des méthodes connues.

Le produit de formule générale (VIII), sous forme d'ester, pour lequel R'₅ représente un radical alcényle éventuellement substitué par un radical phényle peut être obtenu par action d'un acide boronique de formule générale : 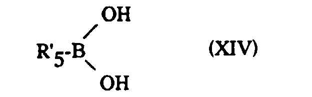 dans laquelle R'₅ est défini comme ci-dessus sur un produit de formule générale (I) dans laquelle R' représente un radical alcoyle éventuellement substitué par un radical phényle.

Généralement, la réaction s'effectue en présence d'un catalyseur tel que le palladium associé à un ligand tel que la triphénylphosphine, en opérant dans un solvant organique tel qu'un hydrocarbure aromatique (benzène, toluène, xylène) à une température comprise entre 0 et 100°C.

Le produit de formule générale (VIII), sous forme d'ester, pour lequel R'₅ représente un radical alcynyle peut être obtenu par action d'un dérivé acétylénique de formule générale :

H-C≡C-Si(R")₃ (XV)

dans laquelle R" représente un radical alcoyle contenant 1 à 4 atomes de carbone, sur un produit de formule générale (I) dans laquelle R' représente un radical alcoyle éventuellement substitué par un radical phényle.

Généralement la réaction s'effectue d'abord en présence d'un catalyseur constitué de palladium associé à un ligand tel que la triphénylphosphine et d'un sel de cuivre tel que l'iodure cuivreux en opérant dans un solvant organique basique tel que la diéthylamine à une température voisine de 20°C, puis en présence d'un agent de désilylation tel que le nitrate d'argent dans un solvant protique, de nitrate d'argent dans un solvant organique tel qu'un alcool aliphatique comme l'éthanol de façon à déplacer le reste silylé.

Le produit de formule générale (VIII), sous forme d'ester, pour lequel R'₅ représente un radical formyle, acyle ou aroyle peut être obtenu par ozonolyse d'un produit de formule générale (VIII), sous forme d'ester, dans laquelle R'₅ représente un radical alcényle éventuellement substitué par un radical phényle.

L'ozonolyse est effectuée généralement dans un solvant organique tel qu'un mélange dichlorométhane-méthanol à une température inférieure à -50°C.

Le produit de formule générale (VIII), sous forme d'ester, pour lequel R'₅ représente un radical hydroxyméthyle peut être obtenu par réduction, au moyen d'un cyanoborohydrure alcalin, d'un produit de formule générale (VIII) pour lequel R'₅ représente un radical formyle.

Le produit de formule générale (VIII), sous forme d'ester, pour lequel R'₅ représente un radical carboxy peut être obtenu par oxydation, par exemple au moyen de perborate de sodium, d'un produit de formule générale (VIII) dans laquelle R'₅ représente un radical formyle.

Le produit de formule générale (VIII), sous forme d'ester, pour lequel R'₅ représente un radical alcoxycarbonyle peut être obtenu, par exemple, par action d'un N,N-diméthylformamide acétal, sur un produit de formule générale (VIII) dans laquelle R'₅ représente un radical carboxy.

La saponification d'un produit de formule générale (I) ou d'un produit de formule générale (VIII), sous forme d'ester, dans lesquelles R' représente un radical alcoyle éventuellement substitué par un radical phényle en un produit de formule générale (I) ou un produit de formule générale (VIII) dans lesquelles R' représente un atome d'hydrogène peut être effectuée au moyen d'une base minérale telle que la lithine en opérant dans un milieu hydro-alcoolique tel qu'un mélange eau-méthanol.

Lorsque dans le produit de formule générale (III), R₅ représente un atome d'iode, son remplacement par un radical R'₅ s'effectue dans les conditions décrites précédemment pour transformer un produit de formule générale (I) en produit de formule générale (VIII).

L'estérification de la baccatine III protégée au moyen d'un acide de formule générale (I) ou d'un acide de formule générale (VIII) ou d'un dérivé tel qu'un halogénure, un anhydride ou un anhydride mixte peut être effectuée dans les conditions suivantes :
1) l'estérification au moyen d'un acide de formule générale (I) ou (VIII) peut être effectuée en présence d'un agent de condensation (carbodiimide, carbonate réactif) et d'un agent d'activation (aminopyridine) dans un solvant organique (éthers, esters, cétones, nitriles, hydrocarbures aliphatiques, hydrocarbures aliphatiques halogénés, hydrocarbures aromatiques) à une température comprise entre -10 et 90°C.
2) l'estérification peut aussi être réalisée en utilisant l'acide de formule générale (I) ou (VIII) sous forme d'anhydride en présence d'un agent d'activation (aminopyridine) dans un solvant organique (éthers, esters, cétones, nitriles, hydrocarbures aliphatiques, hydrocarbures aliphatiques halogénés, hydrocarbures aromatiques) à une température comprise entre 0 et 90°C.
3) l'estérification peut aussi être réalisée en utilisant l'acide de formule générale (I) ou (VIII) sous forme d'halogénure ou sous forme d'un anhydride avec un acide aliphatique ou aromatique, éventuellement préparé in situ, en présence d'une base (amine aliphatique tertiaire) en opérant dans un solvant organique (éthers, esters, cétones, nitriles, hydrocarbures aliphatiques, hydrocarbures aliphatiques halogénés, hydrocarbures aromatiques) à une température comprise entre 0 et 80°C.

Le remplacement des groupements protecteurs R₃, R₄, G₁ et G₂ des produits de formules générales (X) ou (XII) par des atomes d'hydrogène peut être effectué en opérant, selon les significations de R₃ et R₄, de la manière suivante :

1) lorsque R₃ représente un atome d'hydrogène ou un radical alcoxy contenant 1 à 4 atomes de carbone ou un radical aryle éventuellement substitué et R₄ représente un atome d'hydrogène, le produit de formule générale (X) ou (XII) est traité en milieu acide pour obtenir un produit de formule générale : 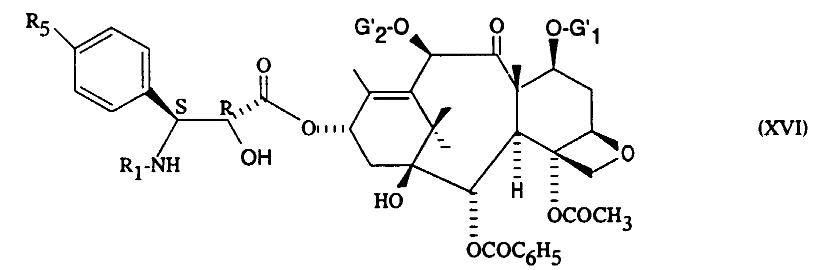 dans laquelle R₁ et R₅ sont définis comme précédemment, G'₁ représente un atome d'hydrogène ou un groupement protecteur de la fonction hydroxy et G'₂ représente un atome d'hydrogène ou un radical acétyle ou un groupement protecteur de la fonction hydroxy, dont les groupements protecteurs G'₁ et G'₂ sont, si nécessaire, remplacés par des atomes d'hydrogène pour obtenir un produit de formule générale (III).

La déprotection de la chaîne latérale du produit de formule générale (X) ou (XII) peut être effectuée en présence d'un acide minéral (acide chlorhydrique, acide sulfurique) ou organique (acide acétique, acide méthanesulfonique, acide trifluorométhanesulfonique, acide p.toluènesulfonique), utilisé seul ou en mélange, en opérant dans un solvant organique choisi parmi les alcools (méthanol, éthanol, isopropanol), les éthers (tétrahydrofuranne, éther diisopropylique, méthyl t.butyléther), les esters (acétate d'éthyle, acétate d'isopropyle, acétate de n.butyle), les hydrocarbures aliphatiques (pentane, hexane, heptane), les hydrocarbures aliphatiques halogénés (dichloro-méthane, dichloro-1,2 éthane), les hydrocarbures aromatiques (benzène, toluène, xylènes) et les nitriles (acétonitrile) à une température comprise entre -10 et 60°C, de préférence entre 15 et 30°C. L'acide peut être utilisé en quantité catalytique, stoechiométrique ou en excès.

La déprotection peut aussi être réalisée dans des conditions oxydantes en utilisant par exemple le nitrate d'ammonium et de cérium IV dans un mélange acétonitrile-eau ou la dichloro-2,3 dicyano-5,6 benzoquinone-1,4 dans l'eau.

La déprotection peut être également réalisée dans des conditions réductrices, par exemple par hydrogénolyse en présence d'un catalyseur.

Les radicaux G₁ et G₂, ainsi que G'₁ et G'₂, lorsqu'ils représentent un groupement protecteur de la fonction hydroxy, sont de préférence des radicaux trichloro-2,2,2 éthoxycarbonyle, (trichlorométhyl-2 propoxy)-2 carbonyle ou des radicaux trialkylsilyles, dialkylarylsilyles, alkyldiarylsilyles ou triarylsilyles dans lesquels les parties alkyles contiennent 1 à 4 atomes de carbone et les parties aryles sont de préférence des radicaux phényles.

Le remplacement par des atomes d'hydrogène, dans le produit de formule générale (XVI), des groupements protecteurs G'₁ et éventuellement G'₂ représentant un radical silylé peut être effectué simultanément avec la déprotection de la chaîne latérale.

Le remplacement par des atomes d'hydrogène, dans le produit de formule générale (XVI), des groupements protecteurs G'₁ et G'₂ représentant un radical trichloro-2,2,2 éthoxycarbonyle ou (trichlorométhyl-2 propoxy)-2 carbonyle est effectué par le zinc, éventuellement associé au cuivre, en présence d'acide acétique à une température comprise entre 20 et 60°C ou au moyen d'un acide minéral ou organique tel que l'acide chlorhydrique ou l'acide acétique en solution dans un alcool aliphatique contenant 1 à 3 atomes de carbone ou dans un ester aliphatique tel que l'acétate d'éthyle, l'acétate d'isopropyle ou l'acétate de n.butyle en présence de zinc éventuellement associé à du cuivre,
2) lorsque R₁ représente un radical t.butoxycarbonyle, R₃ et R₄, identiques ou différents, représentent un radical alcoyle contenant 1 à 4 atomes de carbone, ou un radical aralcoyle dont la partie alcoyle contient 1 à 4 atomes de carbone et la partie aryle est, de préférence un radical phényle, éventuellement substitué, ou un radical aryle de préférence phényle ou bien R₃ représente un radical trihalométhyle ou un radical phényl substitué par un radical trihalométhyle et R₄ représente un atome d'hydrogène, ou bien R₃ et R₄ forment ensemble avec l'atome de carbone auquel ils sont liés un cycle ayant 4 à 7 chaînons, le produit de formule générale (X) ou (XII) est transformé en milieu acide en produit de formule générale (XI) ou (XIII) qui est acylé au moyen de chlorure de benzoyle ou d'un dérivé réactif de formule générale :

R₂-O-CO-X (XVII)

dans laquelle R₂ est défini comme précédemment et X représente un atome d'halogène (fluor, chlore) ou un reste -O-R₂ ou -O-CO-O-R₂, pour obtenir un produit de formule générale (XVI) dont les groupements protecteurs G'₁ et éventuellement G'₂ sont remplacés, si nécessaire, par des atomes d'hydrogène pour obtenir un produit de formule générale (III).

Les produits de formule générale (XI) ou (XIII), dans laquelle G'₁ représente un groupement protecteur de la fonction hydroxy choisi parmi les radicaux trichloro-2,2,2 éthoxycarbonyle et (trichlorométhyl-2 propoxy)-2 carbonyle et G'₂ représente un radical acétyle ou un groupement protecteur de la fonction hydroxy choisi parmi les radicaux trichloro-2,2,2 éthoxycarbonyle et (trichloro-méthyl-2 propoxy)-2 carbonyle, peuvent être obtenus en traitant un produit de formule générale (X) ou (XII), dans laquelle R₁, G₁ et G₂ sont définis comme ci-dessus, R₃ et R₄, identiques ou différents, représentent un radical alcoyle, aralcoyle ou aryle, ou bien R₃ et R₄ forment ensemble avec l'atome de carbone auquel ils sont liés un cycle ayant 4 à 7 chaînons, par un acide minéral (acide chlorhydrique, acide sulfurique) ou organique (acide formique) éventuellement dans un alcool contenant 1 à 3 atomes de carbone (méthanol, éthanol, isopropanol) à une température comprise entre 0 et 50°C. De préférence, on utilise l'acide formique à une température voisine de 20°C.

Les produits de formule générale (XI) ou (XIII), dans laquelle G'₁ représente un atome d'hydrogène et G'₂ représente un radical acétyle peuvent être obtenus en traitant un produit de formule générale (X) ou (XII), dans laquelle G₁ représente un radical silylé et G₂ représente un radical acétyle, R₃ et R₄, identiques ou différents, représentent un radical alcoyle, aralcoyle ou aryle, ou bien R₃ et R₄ forment ensemble avec l'atome de carbone auquel ils sont liés un cycle ayant 4 à 7 chaînons, par un acide minéral (acide chlorhydrique, acide sulfurique, acide fluorhydrique) ou organique (acide formique, acide acétique, acide méthane-sulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C.

Les produits de formule générale (XI) ou (XIII) dans laquelle G'₁ représente un atome d'hydrogène et G'₂ représente un atome d'hydrogène ou un radical acétyle peuvent être obtenus en traitant un produit de formule générale (X) ou (XII), dans laquelle G₁ représente un groupement protecteur choisi parmi les radicaux trichloro-2,2,2 éthoxycarbonyle ou (trichlorométhyl-2 propoxy)-2 carbonyle, G₂ représente un radical acétyle ou un groupement protecteur choisi parmi les radicaux trichloro-2,2,2 éthoxycarbonyle ou (trichlorométhyl-2 propoxy)-2 carbonyle, R₃ représente un radical trihalométhyle ou phényle substitué par un radical trihalométhyle et R₄ représente un atome d'hydrogène, par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone (méthanol, éthanol, propanol, isopropanol) ou dans un ester aliphatique (acétate d'éthyle, acétate d'isopropyle ou acétate de n.butyle) en présence de zinc éventuellement associé à du cuivre.

L'acylation du produit de formule générale (XI) ou (XIII) au moyen de chlorure de benzoyle ou d'un dérivé réactif de formule générale (XVII) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.

Le remplacement éventuel par des atomes d'hydrogène des groupements protecteurs G'₁ et G'₂ du produit de formule générale (XVI), lorsqu'ils représentent un radical trichloro-2,2,2 éthoxycarbonyle ou (trichlorométhyl-2 propoxy)-2 carbonyle, est effectué généralement par traitement par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone (méthanol, éthanol, isopropanol) ou dans un ester aliphatique (acétate d'éthyle, acétate d'isopropyle, acétate de n.butyle) en présence de zinc éventuellement associé à du cuivre.

Les exemples suivants illustrent la présente invention.

### EXEMPLE 1

A une solution de 12,2 g de tert-butoxycarbonyl-3 diméthyl-2,2 phényl-4 oxazolidinecarboxylate-5-(4S,5R) de méthyle dans 130 cm3 de dichlorométhane, maintenue sous atmosphère d'argon et sous agitation, on ajoute, à une température voisine de 20°C, 9,25 g d'iode bisublimée puis par petites quantités, en 5 minutes, 17,2 g de bis-(trifluoroacétoxy)iodobenzène. Le mélange réactionnel est ensuite agité à une température voisine de 25°C pendant 40 minutes puis additionné de 130 cm3 d'une solution aqueuse saturée d'hydrogénocarbonate de sodium et maintenu à nouveau sous agitation pendant 5 minutes. La phase aqueuse décantée est extraite par 2 fois 100 cm3 de dichlorométhane. Les phases organiques sont réunies, lavées par 2 fois 10 cm3 d'eau distillée, séchées sur du sulfate de magnésium, filtrées puis concentrées à sec sous pression réduite (2,7 kPa) à 40°C. On obtient 15,8 g d'une huile jaune que l'on purifie par chromatographie sur 600 g de silice (0,063-0,2 mm) contenus dans une colonne de 7 cm de diamètre en éluant avec un mélange cyclohexane-acétate d'éthyle (75-25 en volumes) en recueillant des fractions de 40 cm3. Les fractions ne contenant que le produit cherché sont réunies et concentrées à sec sous pression réduite (2,7 kPa) à une température voisine de 40°C. On obtient 16,3 g d'une huile jaune que l'on purifie à nouveau par chromatographie sur 900 g de silice (0,063-0,2 mm) contenus dans une colonne de 7 cm de diamètre en éluant avec un mélange cyclohexane-acétate d'éthyle (75-25 en volumes) en recueillant des fractions de 40 cm3. Les fractions ne contenant que le produit cherché sont réunies et concentrées à sec sous pression réduite (2,7 kPa) à 40°C. On obtient ainsi 12,1 g de tert-butoxycarbonyl-3 diméthyl-2,2 (iodo-4 phényl)-4 oxazolidinecarboxylate-5-(4S,5R) de méthyle sous forme d'une huile jaune.

Le tert-butoxycarbonyl-3 diméthyl-2,2 phényl-4 oxazolidinecarboxylate-5-(4S,5R) de méthyle, qui peut être préparé selon la méthode décrite dans la demande PCT WO 9209589 pour la préparation du tert-butoxycarbonyl-3 diméthyl-2,2 phényl-4 oxazolidinecarboxylate-5-(4S,5R) d'éthyle, est obtenu sous forme d'une huile jaune, a un pouvoir rotatoire: [α]²⁰_{D}= -8,6 (c = 1,1; CHCl₃).

### EXEMPLE 2

A une solution de 1,3 g de tert-butoxycarbonyl-3 diméthyl-2,2 (iodo-4 phényl)-4 oxazolidinecarboxylate-5-(4S,5R) de méthyle dans 25 cm3 d'éthanol, on ajoute à une température voisine de 25°C, une solution de 0,35 g d'hydrate d'hydroxyde de lithium dans 8 cm3 d'eau distillée. Le milieu réactionnel est agité pendant 30 minutes à une température voisine de 25°C puis concentré à sec sous pression réduite (2,7 kPa) à une température voisine de 40°C. Le résidu d'évaporation est dissous dans 20 cm3 d'eau distillée et la solution obtenue est lavée par 2 fois 15 cm3 d'oxyde de diéthyle, acidifiée à un pH voisin de 2 par addition d'une solution aqueuse 1N d'acide chlorhydrique et extraite par 2 fois 40cm3 d'acétate d'éthyle. Les phases organiques sont réunies, lavées par 2 fois 10 cm3 d'eau distillée, séchées sur sulfate de magnésium, filtrées puis concentrées à sec sous pression réduite (2,7 kPa) à une température voisine de 40°C. On obtient ainsi 1,3 g d'acide tert-butoxycarbonyl-3 diméthyl-2,2 (iodo-4 phényl)-4 oxazolidinecarboxylique-5-(4S,5R) sous forme d'une huile jaune.

### EXEMPLE 3

A une solution de 0,805 g d'acide tert-butoxycarbonyl-3 diméthyl-2,2 (iodo-4 phényl)-4 oxazolidinecarboxylique-5-(4S,5R) dans 20 cm3 de toluène anhydre on ajoute 0,4 g de N,N'-dicyclohexylcarbodiimide, 1,08 g d'acétoxy-4 benzoyloxy-2α époxy-5β,20 dihydroxy-1,13α oxo-9 bis-(trichloro-2,2,2 éthoxy) carbonyloxy-7β,10β taxène-11 et 0,073 g de diméthylamino-4 pyridine. Le milieu réactionnel est ensuite chauffé sous agitation pendant 3 heures à une température voisine de 80°C, puis refroidi à une température voisine de 20°C et additionné d'un mélange de 25 cm3 de dichlorométhane et de 15 cm3 d'une solution aqueuse saturée d'hydrogénocarbonate de sodium. La phase aqueuse est séparée par décantation puis extraite par 25 cm3 de dichlorométhane. Les phases organiques sont réunies, lavées par 2 fois 10 cm3 d'eau distillée, séchées sur du sulfate de magnésium, filtrées puis concentrées à sec sous pression réduite (2,7 kPa) à 40°C. On obtient 2,1 g d'une meringue blanche que l'on purifie par chromatographie sur 70 g de silice (0,063-0,2 mm) contenus dans une colonne de 2,5 cm de diamètre en éluant avec du dichlorométhane et en recueillant des fractions de 20 cm3. Les fractions ne contenant que le produit cherché sont réunies et concentrées à sec sous pression réduite (2,7 kPa) à une température voisine de 40°C. On obtient ainsi 1,35 g de tert-butoxycarbonyl-3 diméthyl-2,2 (iodo-4 phényl)-4 oxazolidinecarboxylate-5-(4S,5R) d'acétoxy-4, benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Une solution de 1,3 g de tert-butoxycarbonyl-3 diméthyl-2,2 (iodo-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans 20 cm3 d'acide formique est agitée pendant 4 heures à une température voisine de 20°C puis concentrée à sec sous pression réduite (2,7 kPa) à une température voisine de 40°C. Le solide résiduel est dissous dans 75 cm3 de dichlorométhane puis la solution obtenue est lavée successivement par 20 cm3 d'une solution aqueuse saturée de chlorure d'ammonium, par 2 fois 10 cm3 d'eau distillée et concentrée à sec sous pression réduite (2,7 kPa) à une température voisine de 40°C. On obtient 1,2 g d'une meringue blanche que l'on purifie par chromatographie sur 70 g de silice (0,063-0,2 mm) contenus dans une colonne de 2,5 cm de diamètre en éluant avec un mélange dichlorométhane-méthanol (98-2 en volumes) en recueillant des fractions de 20 cm3. Les fractions ne contenant que le produit cherché sont réunies et concentrées à sec sous pression réduite (2,7 kPa) à une température voisine de 40°C. On obtient ainsi 1 g d'amino-3 hydroxy-2 (iodo-4 phényl)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Le tert-butoxycarbonylamino-3 (iodo-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α peut être préparé de la manière suivante :

A une solution de 0,95 g d'amino-3 hydroxy-2 (iodo-4 phényl)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans 20 cm3 de dichlorométhane, maintenue sous atmosphère d'argon et sous agitation, on ajoute 0,074 g d'hydrogénocarbonate de sodium puis goutte à goutte, à une température voisine de 20°C, une solution de 0,216 g de dicarbonate de di.tert-butyle dans 5 cm3 de dichlorométhane. La solution obtenue est agitée pendant 16 heures à une température voisine de 20°C puis additionnée d'un mélange de 10 cm3 d'eau distillée et de 25 cm3 de dichlorométhane. La phase aqueuse est séparée par décantation puis extraite par 20 cm3 de dichlorométhane. Les phases organiques sont réunies, lavées par 2 fois 5 cm3 d'eau distillée, séchées sur sulfate de magnésium, filtrées puis concentrées à sec sous pression réduite (2,7 kPa) à 40°C. On obtient 1 g d'une meringue blanche que l'on purifie par chromatographie sur 90 g de silice (0,063-0,2 mm) contenus dans une colonne de 2,5 cm de diamètre en éluant avec un mélange dichlorométhane-méthanol (99-1 en volumes) en recueillant des fractions de 20 cm3. Les fractions ne contenant que le produit cherché sont réunies et concentrées à sec sous pression réduite (2,7 kPa) à une température voisine de 40°C. On obtient ainsi 0,65 g de tert-butoxycarbonylamino-3 (iodo-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Une solution de 0,5 g de tert-butoxycarbonylamino-3 (iodo-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans un mélange de 10 cm3 de méthanol et de 10 cm3 d'acide acétique est chauffée sous agitation et sous atmosphère d'argon jusqu'à une température voisine de 60°C puis additionnée de 1 g de zinc en poudre. Le mélange réactionnel est ensuite agité pendant 5 minutes à 60°C puis refroidi à une température voisine de 20°C et filtré sur verre fritté garni de célite. Le verre fritté est lavé par 3 fois 10 cm3 de dichlorométhane et les filtrats sont réunis puis concentrés à sec sous pression réduite (2,7 kPa) à une température voisine de 40°C.

Le résidu est additionné de 10 cm3 d'eau distillée et le solide cristallisé est séparé par filtration, lavé par 5 fois 5 cm3 d'eau distillée et séché sous pression réduite (0,27 kPa) à 20°C pendant 16 heures. On obtient 0,40 g d'une meringue blanche que l'on purifie par chromatographie sur 40 g de silice (0,063-0,2 mm) contenus dans une colonne de 2 cm de diamètre en éluant avec un mélange dichlorométhane-méthanol (97-3 en volumes) en recueillant des fractions de 20 cm3. Les fractions ne contenant que le produit cherché sont réunies et concentrées à sec sous pression réduite (2,7 kPa) à 40°C. On obtient 0,35 g d'une meringue blanche que l'on purifie à nouveau par chromatographie sur 70 g de silice (0,063-0,2 mm) contenus dans une colonne de 2,5 cm de diamètre en éluant avec un mélange dichlorométhane-méthanol (97,5-2,5 en volumes) en recueillant des fractions de 20 cm3. Les fractions ne contenant que le produit cherché sont réunies et concentrées à sec sous pression réduite (0,27 kPa) à 40°C pendant 16 heures. On obtient ainsi 0,25 g de tert-butoxycarbonylamino-3 (iodo-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -33 (c = 0,34 ; méthanol)
- spectre de R.M.N.: (400 MHz ; CDCl₃ ; δ en ppm) :
   1,15 (s, 3H : -CH₃ 16 ou 17) ; 1,25 (s, 3H : -CH₃ 16 ou 17) ; 1,35 [s, 9H : -C(CH₃)₃] ; 1,72 (s, 1H : -OH1) ; 1,78 (s, 3H : -CH₃ 19) ; 1,87 [mt, 1H : -(CH)-H 6); 1,88 (s, 3H : -CH₃18) ; 2,28 (ab limite, 2H : -CH₂-14) ; 2,39 (s, 3H : -COCH₃); 2,61 [mt, 1H: -(CH)-H6] ; 3,45 (d, J = 4,5 Hz, 1H : -OH 2') ; 3,92 (d, J = 7 Hz, 1H : -H3) ; 4,20 (d, J = 8 Hz, 1H : -(CH)-H 20) ; 4,23 (s large, 1H : -OH 10) ; 4,25 (mt, 1H : -H7) ; 4,33 [d, J = 8 Hz, 1H : -(CH)-H 20) ; 4,61 (s large, 1H : -H2') ; 4,96 (d large, J = 10 Hz, 1H : -H 5) ; 5,23 (s, 1H : -H10) ; 5,24 (d large, J = 10 Hz, 1H : -H 3') ; 5,42 (d, J = 10 Hz, 1H : -CONH-) ; 5,69 (d, J = 7 Hz, 1H : -H 2) ; 6,25 (t, J = 9 Hz, 1H: -H 13); 7,15 et 7,73 [2d, J = 8 Hz, 2H chacun : -C₆H₅ 3' (-H 2, -H 3, -H5 et -H6)] ; 7,50 [t, J = 7,5 Hz, 2H : -OCOC₆H₅(-H 3 et -H 5)] ; 7,62 [t, J = 7,5 Hz, 1H: -OCOC₆H₅(-H4)]; 8,11 [d, J = 7,5 Hz, 2H : -OCOC₆H₅(-H 2 et -H6)].

L'acétoxy-4 benzoyloxy-2α époxy-5β,20 dihydroxy-1,13α oxo-9 bis-(trichloro-2,2,2 éthoxy)carbonyloxy-7β,10β taxène-11 peut être préparé dans les conditions décrites dans le brevet européen EP 0 336 841.

### EXEMPLE 4

A une solution de 1,43 g de tert-butoxycarbonyl-3 diméthyl-2,2 (iodo-4) phényl-4 oxazolidinecarboxylate-5-(4S,5R) de méthyle dans 6 cm3 de toluène, maintenue sous atmosphère d'argon et sous agitation, on ajoute à une température voisine de 20°C, 0,11 g de tétrakis-triphénylphosphine palladium puis goutte à goutte à une température voisine de 20°C, une solution de 0,44 g d'acide phénylboronique dans 1,5 cm3 de méthanol et une solution de 0,63 g d'hydrogénocarbonate de sodium dans 3 cm3 d'eau distillée. Le mélange réactionnel est ensuite chauffé sous agitation, pendant 6 heures à une température voisine de 80°C, puis refroidi à une température voisine de 20°C et additionné d'un mélange de 100 cm3 d'acétate d'éthyle et de 15 cm3 d'eau distillée. La phase organique est séparée par décantation, lavée par 3 fois 5 cm3 d'eau distillée, séchée sur sulfate de magnésium, filtrée puis concentrée à sec sous pression réduite (2,7 kPa) à 40°C. On obtient 1,6 g d'une meringue blanche que l'on purifie par chromatographie sur 90 g de silice (0,063-0,2 mm) contenus dans une colonne de 2,5 cm de diamètre (éluant : dichlorométhane) en recueillant des fractions de 20 cm3. Les fractions ne contenant que le produit cherché sont réunies et concentrées à sec sous pression réduite (2,7 kPa) à une température voisine de 40°C. On obtient ainsi 1,2 g de tert-butoxycarbonyl-3 diméthyl-2,2 (biphénylyl-4)-4 oxazolidinecarboxylate-5-(4S,5R) de méthyle sous forme d'une huile jaune qui est transformé en acide correspondant dans les conditions décrites dans l'exemple 2.

### EXEMPLE 5

En opérant comme dans l'exemple 3, mais à partir de 0,55 g de tert-butoxycarbonylamino-3 (biphénylyl-4)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α, on obtient 0,306 g de tert-butoxycarbonylamino-3 (biphénylyl-4)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -30 (c = 0,58 ; méthanol)
- spectre de R.M.N.: (400 MHz ; CDCl₃ ; δ en ppm) :
   - 1,15(s, 3H : -CH ₃ 16 ou 17) ; 1,26 (s, 3H : -CH₃ 16 ou 17) ; 1,37 [s, 9H : -C(CH₃)₃] ; 1,74 (s, 1H : -OH 1) ; 1,78 (s, 3H : -CH₃ 19) ; 1,87 [mt, 1H : -(CH)-H 6); 1,90 (s, 3H : -CH₃ 18) ; 2,27 à 2,35 (2dd, J = 17 et 9 Hz, 1H chacun : -CH₂- 14); 2,43 (s, 3H : -COCH₃) ; 2,60 [mt, 1H: -(CH)-H 6] ; 3,48 (d, J = 4,5 Hz, 1H : -OH 2') ; 3,94 (d, J = 7 Hz, 1H : -H3) ; 4,20 (d, J = 8 Hz, 1H : -(CH)-H 20) ; 4,24 (s large, 1H : -OH 10) ; 4,25 (mt, 1H : -H 7) ; 4,33 [d, J = 8 Hz, 1H : -(CH)-H 20] ; 4,69 (s large, 1H : -H2') ; 4,96 (d large, J = 10 Hz, 1H : -H 5) ; 5,23 (s, 1H : -H10) ; 5,34 (d large, J = 10 Hz, 1H : -H 3') ; 5,50 (d, J =10 Hz, 1H : -CONH-) ; 5,69 (d, J = 7 Hz, 1H: -H 2) ; 6,26 (t, J = 9 Hz, 1H : -H13) ; 7,30 à 7,65 (mt, 12H : -H aromatiques) 8,11 [d, J = 7,5 Hz, 2H : -OCOC₆H₅(-H 2 et -H6)].

En opérant comme dans l'exemple 3, à partir de matières premières convenables, sont préparés les intermédiaires suivants :

Le tert-butoxycarbonylamino-3 (biphénylyl-4)-3 hydroxy-2 propionate-(2R,3S) d'acétoxy4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

L'amino-3 hydroxy-2 (biphénylyl-4)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

Le tert-butoxycarbonyl-3 diméthyl-2,2 (biphénylyl-4)-4 oxazolidinecarboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

### EXEMPLE 6

Le tert-butoxycarbonyl-3 diméthyl-2,2 (vinyl-4 phényl)-4 oxazolidinecarboxylate-5-(4S,5R) de méthyle peut être préparé comme décrit à l'exemple 4 pour le tert-butoxycarbonyl-3 diméthyl-2,2 (biphénylyl-4)-4 oxazolidinecarboxylate-5-(4S,5R) de méthyle, mais à partir de 2,4 g de tert-butoxycarbonyl-3 diméthyl-2,2 (iodo-4 phényl)-4 oxazolidinecarboxylate-5-(4S,5R) de méthyle et de 0,75 g d'acide vinylboronique. On obtient ainsi 1,1 g de tert-butoxycarbonyl-3 diméthyl-2,2 (vinyl-4 phényl)-4 oxazolidinecarboxylate-5-(4S,5R) de méthyle sous forme d'une huile jaune qui est transformé en acide correspondant dans les conditions décrites dans l'exemple 2.

L'acide vinylboronique peut être préparé selon la méthode décrite par J. Braun et H. Normant., Bull. Soc. Chim. Fr., 1966 (8), 2257.

### EXEMPLE 7

En opérant comme dans l'exemple 3, mais à partir de 0,84 g de tert-butoxycarbonylamino-3 (vinyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α, on obtient 0,305 g de tert-butoxycarbonylamino-3 (vinyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -36 (c = 0,53 ; méthanol)
- spectre de R.M.N.: (250 MHz ; CDCl₃ . δ en ppm) :
   1,15 (s, 3H : -CH₃ 16 ou 17); 1,26 (s, 3H : -CH₃ 16 ou 17) ; 1,37 (s, 9H : -C(CH₃)₃) ; 1,71 (s, 1H : -OH 1) ; 1,78 (s, 3H : -CH₃ 19) ; 1,85 [mt, 1H : -(CH)-H 6] ; 1,88 (s, 3H : -CH₃ 18) ; 2,30 (ab limite, 2H : -CH₂- 14) ; 2,39 (s, 3H : -COCH₃) ; 2,60 [mt, 1H : -(CH)-H 6) ; 3,41 (mf, 1H : -OH 2'); 3,92 (d, J = 7 Hz, 1H : -H3) ; 4,20 (d, J = 8 Hz, 1H: -(CH)-H 20) ; 4,24 (s large, 1H : -OH en 10) ; 4,25 (mt, 1H : -H 7) ; 4,33 [d, J = 8 Hz, 1H: -(CH)-H 20] ; 4,64 (s large, 1H : -H2') ; 4,96 (d large, J = 10 Hz, 1H: -H 5) ; 5,23 (s, 1H: -H10) ; 5,26 (d large, J = 10 Hz, 1H: -H 3') ; 5,26 d, J = 11 Hz, 1H: -CH=CH(H)(cis)] ; 5,43 (d, J = 10 Hz, 1H : -CONH-) ; 5,69 (d, J = 7 Hz, 1H : -H 2) ; 5,76 (d, J = 17,5 Hz, 1H : -CH=CH(H)(trans)] ; 6,23 (t, J = 9 Hz, 1H : -H 13) 6,70 (dd, J = 17,5 et 11 Hz, 1H: -CH=CH₂) ; 7,35 et 7,44 [2d, J = 8 Hz, 2H chacun : -C₆H₅ en 3' (-H 2, -H 3, -H 5 et -H 6)] ; 7,50 [t, J = 7,5 Hz, 2H : -OCOC₆H₅(-H 3 et -H 5)] ; 7,62 [t, J = 7,5 Hz, 1H : -OCOC₆H₅(-H 4)]; 8,11 [d, J = 7,5 Hz, 2H : -OCOC₆H₅(-H 2 et -H 6)].

En opérant comme dans l'exemple 3, à partir de matières premières convenables, sont préparés les intermédiaires suivants :

Le tert-butoxycarbonylamino-3 (vinyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

L'amino-3 hydroxy-2 (vinyl-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

Le tert-butoxycarbonyl-3 diméthyl-2,2 (vinyl-4 phényl)-4 oxazolidinecarboxylate-5-(45,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

### EXEMPLE 8

Le tert-butoxycarbonyl-3 diméthyl-2,2 (isopropényl-4 phényl)-4 oxazolidinecarboxylate-5-(4S,5R) de méthyle peut être préparé dans les conditions décrites dans l'exemple 1 pour la préparation du tert-butoxycarbonyl-3 diméthyl-2,2 (biphénylyl-4)-4 oxazolidinecarboxylate-5-(4S,5R) de méthyle, mais à partir de 3,0 g de tert-butoxycarbonyl-3 diméthyl-2,2 (iodo-4 phényl)-4 oxazolidinecarboxylate-5-(4S,5R) de méthyle et de 0,81 g d'acide isopropénylboronique. On obtient ainsi 2,07 g de tert-butoxycarbonyl-3 diméthyl-2,2 (isopropényl-4 phényl)-4 oxazolidinecarboxylate-5-(4S,5R) de méthyle sous forme d'une huile jaune qui est transformé en acide correspondant dans les conditions décrites dans l'exemple 2.

L'acide isopropénylboronique peut être préparé selon la méthode décrite par J. Braun et H. Normant., Bull. Soc. Chim. Fr., 1966 (8), 2257.

### EXEMPLE 9

En opérant comme dans l'exemple 3, mais à partir de 1,71 g de tert-butoxycarbonylamino-3 (isoprcpényl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α, on obtient 0,397 g de tert-butoxycarbonylamino-3 (isopropényl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -34 (c = 0,50 ; méthanol)
- spectre de R.M.N.: (400 MHz ; CDCl₃ ; δ en ppm) ;
   1,15 (s, 3H : -CH₃ 16 ou 17) ; 1,26 (s, 3H : -CH₃ 16 ou 17) ; 1,37 [s, 9H : -C(CH₃)₃] ; 1,78 (s, 3H : -CH₃ 19) ; 1,87 [mt, 1H : -(CH)-H 6) ; 1,89 (s, 3H : -CH₃18) ; 2,15 [s, 3H : -C(CH₃)=CH₂] ; 2,26 à 2,34 (2dd, J = 16 et 9 Hz, 1H chacun : -CH₂- 14); 2,41 (s, 3H : -COCH₃) ; 2,60 [mt, 1H : -(CH)-H 6) ; 3,36 (mf, 1H : -OH 2') ; 3,93 (d, J = 7 Hz, 1H : -H3) ; 4,10 à 4,30 (mf, 1H : -OH en 10) ; 4,20 (d, J = 8 Hz, 1H : -(CH)-H 20) ; 4,23 (dd, J = 11 et 6,5 Hz, 1H : -H 7) ; 4,33 [d, J = 8 Hz, 1H : -(CH)-H 20] ; 4,64 (s large, 1H : -H2') ; 4,96 (d large, J = 10 Hz, 1H : -H 5); 5,10 à 5,38 [2s, 1H chacun : -C(CH₃)=CH₂] ; 5,22 (s, 1H : -H10) : 5,28 (d large, J = 10 Hz, 1H : -H 3') ; 5,41 (d, J = 9 Hz, 1H : -CONH-) ; 5,69 (d, J = 7 Hz, 1H : -H 2) ; 6,24 (t, J = 9 Hz, 1H : -H 13) ; 7,35 à 7,50 [2d, J = 7,5 Hz, 2H chacun : -C₆H₅ en 3' (-H 2,-H 3, -H 5 et -H6)] ; 7,51 [t, J= 7,5 Hz, 2H : -OCOC₆H₅(-H 3 et -H 5)] ; 7,62 [t, J = 7,5 Hz, 1H : -OCOC₆H₅ (-H 4)]; 8,11 [d, J = 7,5 Hz, 2H : -OCOC₆H₅(-H 2 et -H6)].

En opérant comme dans l'exemple 3, à partir de matières premières convenables, sont préparés les intermédiaires suivants :

Le tert-butoxycarbonylamino-3 (isopropényl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoiy)carbonyloxy-7β,10β taxène-11 yle-13α sous forme d'une meringue blanche.

L'amino-3 hydroxy-2 (isopropényl-4 phényl)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Le tert-butoxycarbonyl-3 diméthyl-2,2 (isopropényl-4 phényl)-4 oxazolidine-carboxylate-5-(4S,5R) d'acétoxy-4, benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

### EXEMPLE 10

A une solution de 0,07 g de tert-butoxycarbonylamino-3 (vinyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α dans 7 cm3 de dichlorométhane on ajoute 0,1 cm3 de méthanol puis refroidit le milieu réactionnel à une température voisine de -75°C et fait passer pendant 2 heures, à une température voisine de -78°C et sous agitation, un léger courant d'ozone jusquà persistance de la coloration bleue. Le milieu réactionnel est ensuite agité à une température voisine de -75°C pendant 45 minutes en faisant passer un léger courant d'air pour éliminer l'excès d'ozone puis on ajoute 0,1 cm3 de sulfure de diméthyle, réchauffe jusqu'à une température voisine de 20°C et maintient pendant 1 heure à cette température. Le milieu réactionnel est lavé par 2 fois 5 cm3 d'eau distillée, séché sur sulfate de magnésium, filtré puis concentré à sec sous pression réduite (2,7 kPa) à 40°C. On obtient 0,05 g d'une meringue blanche que l'on purifie par chromatographie sur 30 g de silice (0,063-0,2 mm) contenus dans une colonne de 2 cm de diamètre en éluant avec un mélange dichlorométhane-méthanol (97-3 en volumes) en recueillant des fractions de 20 cm3. Les fractions ne contenant que le produit cherché sont réunies et concentrées à sec sous pression réduite (0,27 kPa) à 40°C pendant 16 heures. On obtient ainsi 0,041 g de tert-butoxycarbonylamino-3 (formyl-4 phényl)-3 hydroxy-2 propionate- (2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -26 (c = 0,21; méthanol)
- spectre de R.M.N.: (400 MHz ; CDCl₃ ; δ en ppm) :
   1,16 (s, 3H : -CH₃ 16 ou 17) ; 1,26 (s, 3H : -CH₃ 16 ou 17) ; 1,37 [s, 9H : -C(CH₃)₃] ; 1,78 (s, 3H : -CH₃ 19); 1,87 [mt, 1H : -(CH)-H 6); 1,88 (s, 3H : -CH₃18) ; 2,30 (ab limite, 2H : -CH₂- 14) ; 2,41 (s, 3H : -COCH₃) ; 2,61 [mt, 1H : -(CH)-H 6] ; 3,94 (d, 1H : -H3) ; 4,10 à 4,40 (mf, 1H : -OH en 10) ; 4,20 (d, J = 8 Hz, 1H : -(CH)-H 20) ; 4,24 (dd, J=12 et 7 Hz, 1H : -H 7) ; 4,33 [d,J = 8 Hz, 1H : -(CH)-H 20) ; 4,69 (s large, 1H : -H2') ; 4,96 (d large, J = 10 Hz, 1H : -H 5) ; 5,22 (s, 1H : -H10) ; 5,39 (d large, J = 10 Hz, 1H : -H 3') ; 5,50 (d, J = 10 Hz, 1H : -CONH-) ; 5,69 (d,J = 7 Hz, 1H: -H 2) ; 6,29 (t, J = 9 Hz, 1H: -H 13) ; 7,50 [t, J = 7,5 Hz, 2H : -OCOC₆H₅(-H 3 et -H 5)] ; 7,58 et 7,94 [2d, J = 7,5 Hz, 2H chacun : -C₆H₅ 3' (-H 2, -H 3, -H 5 et -H 6)] ; 7,62 [t, J = 7,5 Hz, 1H : -OCOC₆H_{S} (-H 4)]; 8,12 [d, J = 7,5 Hz, 2H : -OCOC₆H₅(-H 2 et -H 6)] ; 10,04 (s, 1H : -CHO).

### EXEMPLE 11

A une solution de 0,28 g de tert-butoxycarbonylamino-3 (formyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α dans 15 cm3 de méthanol on ajoute simultanément sous agitation, par petites quantités et à une température voisine de 20°C, 0,025 g de cyanotrihydruroborate de sodium et goutte à goutte une solution 2N d'acide chlorhydrique dans l'oxyde de diéthyle afin de maintenir le pH aux environs de 6. Le milieu réactionnel est ensuite agité à une température voisine de 20°C pendant 5 heures puis concentré à sec sous pression réduite (2,7 kPa) à 40°C. On obtient 0,25 g d'une meringue blanche que l'on purifie par chromatographie sur 40 g de silice (0,063-0,2 mm) contenus dans une colonne de 2 cm de diamètre en éluant avec un mélange dichlorométhane-méthanol (97-3 en volumes) en recueillant des fractions de 20 cm3. Les fractions ne contenant que le produit cherché sont réunies et concentrées à sec sous pression réduite (2,7 kPa) à 40°C. On obtient 0,135 g d'une meringue blanche que l'on purifie par chromatographie sur gel de silice déposé sur plaque (gel de 1 cm d'épaisseur ; plaques de 20 x 20 cm) par fractions de 10 mg. Après localisation aux rayons U.V. de la zone correspondant au produit cherché adsorbé, cette zone est grattée et la silice recueillie est lavée sur verre fritté par 10 fois 10 cm3 de dichlorométhane et par 5 fois 5 cm3 de méthanol. Les filtrats sont réunis et concentrés à sec sous pression réduite (0,27 kPa) à 40°C pendant 16 heures. On obtient ainsi 0,077 g de tert-butoxycarbonylamino-3 (hydroxyméthyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -33 (c = 0,51 ; méthanol)
- spectre de R.M.N.: (400 MHz ; CDCl₃ ; δ en ppm)
   1,13 (s, 3H : -CH₃ 16 ou 17) ; 1,22 (s, 3H : -CH₃ 16 ou 17) ; 1,37 (s, 9H : -C(CH₃)₃) ; 1,75 (s, 3H : -CH₃ 19) ; 1,84 [mt, 1H : -(CH)-H 6) ; 1,86 (s, 3H : -CH₃18) ; 2,00 à 2,25 (mt, 2H : -CH₂-14) ; 2,38 (s, 3H : -COCH₃) ; 2,58 [mt, 1H : -(CH)-H 6] ; 3,40 à 3,70 (mf, 1H : -OH 2') ; 3,87 (d, J = 7 Hz, 1H : -H3) ; 4,10 à 4,30 (mf, 1H : -OH 10) ; 4,18 (d, J = 8 Hz, 1H : -(CH)-H 20) ; 4,20 (dd, J = 11 et 6 Hz, 1H : -H 7) ; 4,32 [d, J = 8 Hz, 1H : -(CH)-H 20) ; 4,49 et 4,63 (2d, J = 13 Hz, 1H chacun : -CH₂OH) ; 4,56 (s large, 1H : -H 2') ; 4,95 (d large, J = 10 Hz, 1H : -H 5) ; 5,18 (mt, 1H : -H 3') ; 5,20 (s, 1H : -H 10) ; 5,43 (d, J = 10 Hz, 1H : -CONH-) ; 5,67 (d, J = 7 Hz, 1H : -H 2) ; 6,08 (t, J = 9 Hz, 1H : -H 13) ; 7,30 à 7,45 [mt, 4H : -C₆H₅ 3' (-H 2, -H 3, -H 5 et -H 6)] ; 7,53 [t, J = 7,5 Hz, 2H : -OCOC₆H₅(-H 3 et -H 5)] ; 7,64 [t, J = 7,5 Hz, 1H : -OCOC₆H₅(-H 4)]; 8,12 [d, J = 7,5 Hz, 2H : -OCOC₆H₅(-H 2 et -H6)].

### EXEMPLE 12

A une solution de 0,33 g de tert-butoxycarbonylamino-3 (isopropényl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α dans 15 cm3 de dichlorométhane on ajoute 0,07 cm3 de méthanol puis refroidit le milieu réactionnel à une température voisine de -65°C et fait passer pendant 3 heures, à une température voisine de -65°C et sous agitation, un léger courant d'ozone jusquà persistance de la coloration bleue. Le milieu réactionnel est ensuite agité à une température voisine de -65°C pendant 1 heure en faisant passer un léger courant d'air pour éliminer l'excès d'ozone puis on ajoute 0,26 cm3 de sulfure de diméthyle, réchauffe jusqu'à une température voisine de 20°C et maintient pendant 30 minutes à cette température. Le milieu réactionnel est lavé par 2 fois 10 cm3 d'eau distillée, séché sur sulfate de magnésium, filtré puis concentré à sec sous pression réduite (2,7 kPa) à 40°C. On obtient 0,30 g d'une huile jaune que l'on purifie par chromatographie sur 20 g de silice (0,063-0,2 mm) contenus dans une colonne de 1,5 cm de diamètre en éluant avec un mélange dichlorométhane-méthanol (99-1 en volumes) en recueillant des fractions de 10 cm3. Les fractions ne contenant que le produit cherché sont réunies et concentrées à sec sous pression réduite (2,7 kPa) à 40°C. On obtient ainsi 0,25 g de tert-butoxycarbonylamino-3 (acétyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une huile jaune.

### EXEMPLE 13

En opérant comme dans l'exemple 3, mais à partir de 0,54 g de tert-butoxycarbonylamino-3 (acétyl-4 phényl)-3 hydroxy-2 prupionate-(2R,3S) d'acétoxy-4 benzoyloxy-2α époxy-5β,20 hydroxy-1 oxo-9 bis-(trichloro-2,2,2 éthoxy)carbonyl oxy-7β,10β taxène-11 yle-13α, on obtient 0,205 g de tert-butoxycarbonylamino-3 (acétyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -33 (c = 0,53 ; méthanol)
- spectre de R.M.N.: (400 MHz ; CDCl₃ ; δ en ppm) _{,}
   1,15 (s, 3H : -CH₃ 16 ou 17) ; 1,25 (s, 3H : -CH₃ 16 ou 17); ; 1,35 (s, 9H : -C(CH₃)₃) ; 1,78 (s, 3H : -CH₃ 19) ; 1,87 [mt, 1H : -(CH)-H 6) ; 1,89 (s, 3H : -CH₃18) ; 2,30 (ab limite, 2H : -CH₂- 14); 2,40 (s, 3H : -COCH₃) ; 2,60 [mt, 1H : -(CH)-H 6) ; 2,63 [s, 3H : -C₆H₄ (p-COCH₃)] ; 3,93 (d, J = 7 Hz, 1H : -H 3) ; 4,20 (d, J = 8 Hz, 1H : -(CH)-H 20) ; 4,24 (dd, J = 11 et 6 Hz, 1H : -H 7) ; 4,33 (d, J = 8 Hz, 1H : -(CH)-H 20) ; 4,67 (s large, 1H : -H 2') ; 4,96 (d large, J = 10 Hz, 1H : -H 5); 5,22 (s, 1H : -H 10) ; 5,35 (d large, J = 10 Hz, 1H : -H 3') ; 5,53 (d, J = 10 Hz, 1H: -CONH-) ; 5,69 (d, J = 7 Hz, 1H : -H 2) ; 6,27 (t, J = 9 Hz, 1H : -H 13) ; 7,50 [t, J = 7,5 Hz, 2H : -OCOC₆H₅(-H 3 et -H 5)] ; 7,51 et 8,00 [2d, J = 7,5 Hz, 2H chacun: -C₆H₅ 3' (-H 2, -H 3, -H 5 et -H 6)] ; 7,62 [t, J = 7,5 Hz, 1H : -OCOC₆H₅ (-H 4)]; 8,11 [d, J = 7,5 Hz, 2H : -OCOC₆H₅(-H 2 et -H 6)].

En opérant comme dans l'exemple 3, à partir de matières premières convenables, sont préparés les intermédiaires suivants :

Le tert-butoxycarbonylamino-3 (acétyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

L'amino-3 hydroxy-2 (acétyl-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

Le tert-butoxycarbonyl-3 diméthyl-2,2 (acétyl-4 phényl)-4 oxazolidinecarboxylate-5-(4S,5R) d'acétoxy-4, benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

### EXEMPLE 14

A une solution de 2,13 g de tert-butoxycarbonyl-3 diméthyl-2,2 (iodo-4) phényl-4 oxazolidinecarboxylate-5-(4S,5R) de méthyle dans 25 cm3 de diéthylamine, maintenue sous atmosphère d'argon et sous agitation, on ajoute à une température voisine de 20°C, 0,12 g de tétrakis-triphénylphosphine palladium puis 0,933 cm3 de triméthysilylacétylène et 5 mg de iodure cuivreux. Le mélange réactionnel est ensuite agité à une température voisine de 20°C pendant 16 heures puis concentré à sec sous pression réduite (2,7 kPa) à 40°C. Le résidu d'évaporation est dissous dans 150 cm3 d'acétate d'éthyle et la solution obtenue est additionnée de 1 g de noir végétal, agitée pendant 10 minutes, filtrée puis concentrée à sec sous pression réduites (2,7 kPa) à 40°C. L'huile résiduelle est à nouveau dissoute dans 150 cm3 d'acétate d'éthyle et la solution obtenue est lavée par 5 fois 15 cm3 d'eau distillée, additionnée de 1 g de noir végétal, agitée pendant 10 minutes, filtrée puis concentrée à sec sous pression réduite (2,7 kPa) à 40°C. On obtient 2,2 g d'une meringue blanche que l'on purifie par chromatographie sur 90 g de silice (0,063-0,2 mm) contenus dans une colonne de 3 cm de diamètre (éluant : dichlorométhane) en recueillant des fractions de 20 cm3. Les fractions ne contenant que le produit cherché sont réunies et concentrées à sec sous pression réduite (2,7 kPa) à une température voisine de 40°C. On obtient ainsi 1,45 g de tert-butoxycarbonyl-3 diméthyl-2,2 (triméthylsilyléthynyl-4)-4 oxazolidinecarboxylate-5-(4S,5R) de méthyle sous forme d'une huile jaune.

A une solution de 1,4 g de tert-butoxycarbonyl-3 diméthyl-2,2 (triméthylsilyléthynyl-4 phényl)-4 oxazolidinecarboxylate-5-(4S,5R) de méthyle dans 20 cm3 d'éthanol, on ajoute goutte à goutte sous agitation et à une température voisine de 20 C, une solution de 1,7 g de nitrate d'argent dans un mélange de 2 cm3 d'eau distillée et de 1 cm3 d'éthanol. Le mélange réactionnel est ensuite agité pendant 2 heures à une température voisine de 20°C puis on additionne, goutte à goutte, une solution de 2,93 g de cyanure de potassium dans 3 cm3 d'eau distillée, maintient l'agitation à une température voisine de 20°C pendant 18 heures et extrait par 3 fois 50 cm3 d'acétate d'éthyle. Les phases organiques sont réunies, lavées par 3 fois 5 cm3 d'eau distillée, séchées sur sulfate de magnésium, filtrées puis concentrées à sec sous pression réduite (2,7 kPa) à 40°C. On obtient ainsi 0,55 g de tert-butoxycarbonyl-3 diméthyl-2,2 (éthynyl-4 phényl)-4 oxazolidinecarboxylate-5-(4S,5R) de méthyle sous forme d'une huile brune qui est transformé en acide correspondant dans les conditions décrites dans l'exemple 2.

### EXEMPLE 15

En opérant comme dans l'exemple 3, mais à partir de 0,63 g de tert-butoxycarbonylamino-3 (éthynyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α, on obtient 0,288 g de tert-butoxycarbonylamino-3 (éthynyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -35° (c = 0,56 ; méthanol)
- spectre de R.M.N.: (400 MHz ; CDCl₃ ; δ en ppm)
   1,15 (s, 3H : -CH₃ 16 ou 17) ; 1,26 (s, 3H : -CH₃ 16 ou 17); 1,37 (s, 9H : -C(CH₃)₃) ; 1,70 (s large, 1H : -OH 1) ; 1,78 (s, 3H : -CH₃ 19) ; 1,87 [mt, 1H : -(CH)-H 6) ; 1,89 [mt, 3H : -CH₃18) ; 2,29 (ab limite, 2H : -CH₂- 14); 2,38 (s, 3H : -COCH₃) ; 2,60 [mt, 1H : -(CH)-H 6] ; 3,10 (s, 1H : -C=CH) ; 3,42 (s large, 1H : -OH 2') ; 3,93 (d, J = 7 Hz, 1H : -H 3) ; 4,20 (d, J = 8 Hz, 1H,: -(CH)-H 20) ; 4,23 (mt, 2H : -H 7 et -OH 10) ; 4,33 [d, J = 8 Hz, 1H : -(CH)-H 20) ; 4,64 (s large, 1H : -H 2') ; 4,96 (d large, J = 10 Hz, 1H: -H 5) ; 5,22 (s, 1H : -H 10) ; 5,28 (d large J = 10 Hz, 1H: -H 3') ; 5,43 (d, J = 10 Hz, 1H : -CONH-) ; 5,69 (d, J = 7 Hz, 1H : -H 2) ; 6,25 (t, J = 9 Hz, 1H : -H 13) ; 7,36 et 7,53 [2d, J = 7,5 Hz, 2H chacun : -C₆H₅ 3' (-H 2, -H 3, -H 5 et -H 6)] ; 7,51 [t, J = 7,5 Hz, 2H : -OCOC₆H₅(-H 3 et -H 5)] ; 7,62 [t, J = 7,5 Hz, 1H : -OCOC₆H₅(-H 4)] ; 8,11 [d, J = 7,5 Hz,2H : -OCOC₆H₅(-H 2 et -H 6)].

En opérant comme dans l'exemple 3, à partir de matières premières convenables, sont préparés les intermédiaires suivants :

Le tert-butoxycarbonylamino-3 (éthynyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

L'amino-3 hydroxy-2 (éthynyl-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

Le tert-butoxycarbonyl-3 diméthyl-2,2 (éthynyl-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

### EXEMPLE 16

A une solution de 6 g de tert-butoxycarbonyl-3 diméthyl-2,2 (vinyl-4 phényl)-4 oxazolidinecarboxylate-5-(4S,5R) de méthyle dans 150 cm3 de dichlorométhane on ajoute 1,34 cm3 de méthanol puis refroidit le milieu réactionnel à une température voisine de -74°C et fait passer pendant 2 heures, à une température voisine de -74°C et sous agitation, un léger courant d'ozone jusqu'à persistance de la coloration bleue. Le milieu réactionnel est ensuite agité à une température voisine de -74°C pendant 1 heure en faisant passer un léger courant d'air pour éliminer l'excès d'ozone puis on ajoute 4,9 cm3 de sulfure de diméthyle, réchauffe jusqu'à une température voisine de 20°C et maintient pendant 1 heure à cette température. Le milieu réactionnel est lavé par 3 fois 30 cm3 d'eau distillée, séché sur sulfate de magnésium, filtré puis concentré à sec sous pression réduite (2,7 kPa) à 40°C. On obtient 6,45 g d'une huile jaune que l'on purifie par chromatographie sur 130 g de silice (0,063-0,2 mm) contenus dans une colonne de 3 cm de diamètre (éluant : dichlorométhane) en recueillant des fractions de 10 cm3. Les fractions ne contenant que le produit cherché sont réunies et concentrées à sec sous pression réduite (2,7 kPa) à 40°C. On obtient ainsi 1,99 g de tert-butoxycarbonyl-3 diméthyl-2,2 (formyl-4 phényl)-4 oxazolidinecarboxylate-5-(4S,5R) de méthyle sous forme d'une huile jaune.

A une solution de 2,1 g de tert-butoxycarbonyl-3 diméthyl-2,2 (formyl-4 phényl)-4 oxazolidinecarboxylate-5-(4S,5R) de méthyle dans 25 cm3 d'acide acétique on ajoute 1,06 g d'hydrate de perborate de sodium. Le milieu réactionnel est chauffé sous agitation jusqu'à une température voisine de 45°C, maintenu pendant 10 heures à cette température puis refroidi à une température voisine de 20°C et concentré à sec sous pression réduite (2,7 kPa) à 40°C. L'huile résiduelle est dissoute dans 75 cm3 d'acétate d'éthyle et la solution obtenue est lavée par 5 fois 5 cm3 d'eau distillée, séchée sur sulfate de magnésium, filtrée puis concentrée à sec sous pression réduite (2,7 kPa) à 40°C. On obtient ainsi 2,2 g de tert-butoxycarbonyl-3 diméthyl-2,2 (carboxy-4 phényl)-4 oxazolidinecarboxylate-5-(4S,5R) de méthyle sous forme dune huile jaune.

Une solution de 2,15 g de tert-butoxycarbonyl-3 diméthyl-2,2 (carboxy-4 phényl)-4 oxazolidinecarboxylate-5-(4S,5R) de méthyle dans 45 cm3 de toluène anhydre est chauffée sous agitation et sous atmosphère d'argon jusqu'à une température voisine de 80°C puis additionnée, goutte à goutte en 30 minutes, de 6cm3 de N,N diméthylformamide di-tert-butylacétal. Le milieu réactionnel est ensuite agité à une température voisine de 80°C pendant 2 heures puis refroidi à une température voisine de 20°C, additionné de 100 cm3 d'acétate d'éthyle, lavé par 2 fois 25 cm3 d'une solution aqueuse saturée d'hydrogénocarbonate de sodium puis par 2 fois 10 cm3 d'eau distillée, séché sur sulfate de magnésium, filtré puis concentré à sec sous pression réduite (2,7 kPa) à 40°C. On obtient 2,5 g d'une huile jaune que l'on purifie par chromatographie sur 80 g de silice (0,063-0,2 mm) contenus dans une colonne de 2,5 cm de diamètre (éluant : dichlorométhane) en recueillant des fractions de 20 cm3. Les fractions ne contenant que le produit cherché sont réunies et concentrées à sec sous pression réduite (2,7 kPa) à une température voisine de 40°C. On obtient ainsi 2,1 g de tert-butoxycarbonyl-3 diméthyl-2,2 (tert-butoxycarbonyl-4 phényl)-4 oxazolidinecarboxylate-5-(4S,5R) de méthyle sous forme d'une huile jaune.

### EXEMPLE 17

En opérant comme dans l'exemple 3, mais à partir de 0,485 g de tert-butoxycarbonylamino-3 (tert-butoxycarbonyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α, on obtient 0,136 g de tert-butoxycarbonylamino-3 (tert-butoxycarbonyl-4 phényl)-3 hydroxy-2 propionate-(2R,3S) d'acétoxy-4 benzoyloxy-2α époxy-5β,20 trihydroxy-1,7β,10β oxo-9 taxène-11 yle-13α sous forme d'une meringue blanche dont les caractéristiques sont les suivantes :
- pouvoir rotatoire : [α]²⁰_{D}= -35 (c = 0,46 ; méthanol)
- spectre de R.M.N.: (400 MHz ; CDCl₃ ; δ en ppm)
   1,16 (s, 3H : -CH₃ 16 ou 17) ; 1,28 (s, 3H : -CH₃ 16 ou 17) ; 1,37 [s, 9H : -NHCOOC(CH₃)₃] ; 1,63 [s, 9H : -C₆H₄ (p--COOC(CH₃)₃] ; 1,69 (s, 1H : -OH 1) ; 1,78 (s, 3H : -CH₃ 19) ; 1,87 [mt, 1H : -(CH)-H 6] ; 1,91 (s, 3H : -CH₃18) ; 2,32 (ab limite, 2H : -CH₂- 14); 2,38 (s, 3H : -COCH₃) ; 2,60 [mt, 1H : -(CH)-H 6) ; 3,38 (s large, 1H : -OH 2') ; 3,95 (d, J = 7 Hz, 1H : -H 3) ; 4,17 (s large, 1H : -OH 10) ; 4,22 (d, J = 8 Hz, 1H : -(CH) -H 20) ; 4,23 (mt, 1H : -H 7) ; 4,32 [d, J = 8 Hz, 1H : -(CH)-H 20) ; 4,65 (s large, 1H : -H 2') ; 4,95 (d large, J = 10 Hz, 1H: -H 5) ; 5,22 (s, 1H : -H10) ; 5,34 (d large, J = 10 Hz, 1H : -H 3') ; 5,42 (d, J = 10 Hz, 1H : -CONH-); 5,71 (d, J = 7 Hz, 1H: -H 2) ; 6,28 (t, J = 9 Hz, 1H: -H 13) ; 7,46 et 8,01 [2d, J = 8 Hz, 2H chacun :-C₆H₅ 3' (-H 2, -H 3, -H 5 et -H 6)]; 7,51 [t, J = 7,5 Hz, 2H : -OCOC₆H₅(-H 3 et -H 5)] ; 7,64 [t, J = 7,5 Hz, 1H : -OCOC₆H₅ (-H 4)]; 8,11 [d, J = 7,5 Hz, 2H : -OCOC₆H₅(-H 2 et -H6)].

En opérant comme dans l'exemple 3, à partir de matières premières convenables, sont préparés les intermédiaires suivants :

Le tert-butoxycarbonylamino-3 (tert-butoxycarbonyl-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

L'amino-3 hydroxy-2 (tert-butoxycarbonyl-4 phényl)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Le tert-butoxycarbonyl-3 diméthyl-2,2 (tert-butoxycarbonyl-4 phényl)-4 oxazolidinecarboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche

### EXEMPLE 18

Une solution de 0,55 g de tert-butoxycarbonylamino-3 (carboxy-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans un mélange de 9 cm3 de méthanol et de 9 cm3 d'acide acétique est chauffée sous agitation et sous atmosphère d'argon jusqu'à une température voisine de 60°C puis additionnée de 1,1 g de zinc en poudre. Le mélange réactionnel est ensuite agité pendant 30 minutes à 60°C puis refroidi à une température voisine de 20°C et filtré sur verre fritté garni de célite. Le verre fritté est lavé par 3 fois 10 cm3 de diclorométhane et les filtrats sont réunis puis concentrés à sec sous pression réduite (2,7 kPa) à une température voisine de 40°C. Le résidu est additionné de 10 cm3 d'eau distillée et le solide cristallisé est séparé par filtration, lavé par 5 fois 5 cm3 d'eau distillée et séché sous pression réduite (0,27 kPa) à 20°C pendant 16 heures. On obtient 0,88 g d'une meringue blanche que l'on purifie par chromatographie sur 30 g de silice (0,063-0,2 mm) contenus dans une colonne de 1,5 cm de diamètre en éluant avec un mélange dichlorométhane-méthanol (97-3 en volumes) et en recueillant des fractions de 10 cm3. Les fractions ne contenant que le produit cherché sont réunies et concentrées à sec sous pression réduite (2,7 kPa) à 40°C. On obtient 0,26 g d'une meringue blanche que l'on purifie par chromatographie sur gel de silice déposé sur plaque [épaisseur 0,5 mm ; 7 plaques de 20 x 20 cm ; éluant : dichlorométhane-méthanol (80-20 en volumes)]. Après localisation aux rayons U.V. de la zone correspondant au produit cherché adsorbé, cette zone est grattée et la silice recueillie est lavée sur verre fritté par 10 fois 10 cm3 de dichlorométhane et par 5 fois 5 cm3 de méthanol. Les filtrats sont réunis et concentrés à sec sous pression réduite (0,27 kPa) à 40°C pendant 16 heures. On obtient ainsi 0,177 g d'une meringue blanche que l'on purifie par chromatographie sur phase inverse Whatman KC₁₈F déposée sur plaque [épaisseur 0,2 mm; 10 plaques de 20 x 20 cm, éluant : méthanol-eau (50-50 en volumes)]. Après localisation aux rayons U.V. de la zone correspondant au produit cherché adsorbé, cette zone est grattée et la phase inverse recueillie est lavée sur verre fritté par 10 fois 10 cm3 de dichlorométhane et par 5 fois 5 cm3 de méthanol. Les filtrats sont réunis et concentrés à sec sous pression réduite (0,27 kPa) à 40°C pendant 16 heures. On obtient ainsi 0,074 g de tert-butoxycarbonylamino-3 (carboxy-4 phényl)-3 hydroxy-2 propionate-(2R,3S) d'acétoxy-4 benzoyloxy-2α époxy-5β,20 kihydrcxy-1,7β,10β oxo-9 taxène-11 yle-13 sous forme d'une meringue blanche dont les caractéristiques sont les suivantes :
- pouvoir rotatoire : [α]²⁰_{D}= -41 (c = 0,5 ; méthanol)
- spectre de R.M.N.: (600 MHz ; CDCl₃ ; δ en ppm) : 1,04 (s, 3H : -CH₃ 16 ou 17);
   1,13 (s, 3H : -CH₃ 16 ou 17) ; 1,29 (s, 9H : -C(CH₃)₃) ; 1,64 (s, 3H : -CH₃ 19) ; 1,79 (s, 3H : -CH₃ 18) ; 1,79 et 2,42 (2 mt, 1H chacun : -CH₂ 6) ; 2,10 à 2,25 (mt, 2H : -CH₂ 14); 2,30 (s, 3H : -COCH₃) ; 3,78 (d, J = 7 Hz, 1H : -H 3) ; 4,10 (dd, J = 11 et 7 Hz, 1H : -H 7) ; 4,13 et 4,21 (2d, J = 8,5 Hz, 1H chacun : -CH₂ 20) ; 4,52 (mt, 1H : -H 2'); 4,88 (d large, J = 10 Hz, 1H : -H 5) ; 5,15 (s, 1H : -H10) ; 5,17 (mt, 1H : -H 3') ; 5,58 (d, J = 7 Hz, 1H : -H 2) ; 6,10 (t, J = 9 Hz, 1H : -H13) ; 6,20 (d, J = 10 Hz, 1H : -CONH-) ; 7,37 [d, J = 8 Hz, 2H : -C₆H₅ 3'(-H 3 et H 5)] ; 7,40 [t, J = 7,5 Hz, 2H : -OCOC₆H₅(-H 3 et H 5)] ; 7,52 [t, J = 7,5 Hz, 1H : -OCOC₆H₅(-H 4)] ; 7,96 [t, J = 7,5 Hz, 2H : -OCOC₆H₅(-H 2 et -H 6)] ; 8,05 [d, J = 8 Hz, 2H : -C₆H₅ 3'(-H 2 et -H 6)].

Le tert-butoxycarbonylamino-3 (carboxy-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α peut être préparé de la manière suivante :

A une solution de 1,2 g d'amino-3 hydroxy-2 (carboxy-4 phényl)-3 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α dans 20 cm3 de pyridine, maintenue sous atmosphère d'argon et sous agitation, on ajoute goutte à goutte, à une température voisine de 20°C, une solution de 0,347 g de carbonate de tert-butyle et de tétrachloro-1,2,2,2 éthyle dans 2 cm3 de pyridine. La solution obtenue est agitée pendant 24 heures à une température voisine de 20°C puis additionnée de 200 cm3 de dichlorométhane, lavée par 4 fois 10 cm3 d'eau distillée, séchée sur sulfate de magnésium, filtrée puis concentrée à sec sous pression réduite (2,7 kPa) à 40°C. On obtient 1,67 g d'une meringue blanche que l'on purifie par chromatographie sur 50 g de silice (0,063-0,2 mm) contenus dans une colonne de 2 cm de diamètre en éluant avec un mélange dichlorométhane-méthanol (95-5 en volumes) et en recueillant des fractions de 10 cm3. Les fractions ne contenant que le produit cherché sont réunies et concentrées à sec sous pression réduite (2,7 kPa) à une température voisine de 40°C. On obtient ainsi 0,95 g d'une meringue blanche que l'on purifie par chromatographie sur 22 g de silice (0,063-0,2 mm) contenus dans une colonne de 2 cm de diamètre en éluant avec un mélange dichlorométhane-méthanol (99-1 en volumes) et en recueillant des fractions de 10 cm3. Les fractions ne contenant que le produit cherché sont réunies et concentrées à sec sous pression réduite (2,7 kPa) à une température voisine de 40°C. On obtient ainsi 0,35 g de tert-butoxycarbonylamino-3 (carboxy-4 phényl)-3 hydroxy-2 propionate-(2R,3S) d'acétoxy-4 benzoyloxy-2α époxy-5β,20 hydroxy-1 oxo-9 bis-(trichloro-2,2,2 éthoxy)carbonyloxy-7β,10β taxène-11 yle-13α sous forme d'une meringue blanche.

Le carbonate de tert-butyle et de tétrachloro-1,2,2,2 éthyle peut être préparé selon la méthode décrite par G. Barcelo et al., Synthesis., 1986, 627-632.

L'amino-3 hydroxy-2 (carboxy-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α peut être préparé de la manière suivante :

Une solution de 2,1 g de tert-butoxycarbonyl-3 diméthyl-2,2 [α-méthyl (méthoxy-4) benzyloxycarbonyl] -4 oxazolidinecarboxylate-5- (4S,5R) d'acétoxy-4, benzoyloxy-2α époxy-5β,20 hydroxy-1 oxo-9 bis-(trichloro-2,2,2 éthoxy) carbonyloxy-7β,10β taxène-11 yle-13α dans 20 cm3 d'acide formique est agitée pendant 5 heures à une température voisine de 20°C puis concentrée à sec sous pression réduite (2,7 kPa) à une température voisine de 40°C. Le solide résiduel est dissous dans 200 cm3 d'acétate d'éthyle et la solution obtenue est lavée par 10 cm3 d'une solution aqueuse saturée de chlorure de sodium, puis par 3 fois 10 cm3 d'eau distillée, séchée sur sulfate de magnésium et concentrée à sec sous pression réduite (2,7 kPa) à une température voisine de 40°C. On obtient 1,8 g d'une meringue blanche que l'on purifie par chromatographie sur 54 g de silice (0,063-0,2 mm) contenus dans une colonne de 2,5 cm de diamètre en éluant avec un mélange dichlorométhane-méthanol (95-5 en volumes) et en recueillant des fractions de 20 cm3. Les fractions ne contenant que le produit cherché sont réunies et concentrées à sec sous pression réduite (2,7 kPa) à une température voisine de 40°C. On obtient ainsi 1,23 g d'amino-3 hydroxy-2 (carboxy-4 phényl)-3 propionate-(2R,3S) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

Le tert-butoxycarbonyl-3 diméthyl-2,2 [α-méthyl (méthoxy-4) benzyloxycarbonyl]-4 oxazolidinecarboxylate-5-(4S,5R) d'acétoxy-4 benzoyloxy-2α époxy-5β,20 hydroxy-1 oxo-9 bis-(trichloro-2,2,2 éthoxy)carbonyloxy-7β,10β taxène-11 yle-13α peut être préparé de la manière suivante :

A une solution de 1,7 g d'acide tert-butoxycarbonyl-3 diméthyl-2,2 [α-méthyl (méthoxy-4) benzyloxycarbonyl]-4 oxazolidinecarboxylique-5-(4S,5R) dans 160 cm3 de toluène anhydre on ajoute 1,17 g de N,N'-dicyclohexylcarbodiimide, 2,54g d'acétoxy-4 benzoyloxy-2α époxy-5β,20 dihydroxy-1,13α oxo-9 bis-(trichloro-2,2,2 éthoxy)carbonyloxy-7β,10β taxène-11 et 0,173 g de diméthylamino-4 pyridine. Le milieu réactionnel est ensuite maintenu sous agitation pendant 24 heures à une température voisine de 20°C puis additionné d'un mélange de 25 cm3 de dichlorométhane et de 10 cm3 d'une solution aqueuse saturée d'hydrogénocarbonate de sodium. La phase aqueuse est séparée par décantation puis extraite par 25 cm3 de dichlorométhane. Les phases organiques sont réunies, lavées par 2 fois 10 cm3 d'eau distillée, séchées sur du sulfate de magnésium, filtrées puis concentrées à sec sous pression réduite (2,7 kPa) à 40°C. La meringue blanche obtenue est purifiée par chromatographie sur 200 g de silice (0,063-0,2 mm) contenus dans une colonne de 4,5 cm de diamètre en éluant avec un mélange dichlorométhane-méthanol (99,5-0,5 en volumes) et en recueillant des fractions de 20 cm3. Les fractions ne contenant que le produit cherché sont réunies et concentrées à sec sous pression réduite (2,7 kPa) à une température voisine de 40°C. On obtient ainsi 3,9 g d'une meringue blanche que l'on purifie par chromatographie sur 120 g de silice (0,063-0,2 mm) contenus dans une colonne de 3 cm de diamètre en éluant avec un mélange cyclohexane-acétate d'éthyle (90-10 en volumes) et en recueillant des fractions de 10 cm3. Les fractions ne contenant que le produit cherché sont réunies et concentrées à sec sous pression réduite (2,7 kPa) à une température voisine de 40°C. On obtient ainsi 2,3 g de tert-butoxycarbonyl-3 diméthyl-2,2 [α-méthyl (méthoxy-4) benzyloxycarbonyl]-4 oxazolidinecarboxylate-5-(4S,5R) d'acétoxy-4 benzoyloxy-2α époxy-5β,20 hydroxy-1 oxo-9 bis-(trichloro-2,2,2 éthoxy) carbonyloxy-7β,10β taxène-11 yle-13α sous forme d'une meringue blanche.

L'acétoxy-4 benzoyloxy-2α époxy-5β,20 dihydroxy-1,13α oxo-9 bis-(trichloro-2,2,2 éthoxy)carbonyloxy-7β10β taxène-11 peut être préparé selon la méthode décrite dans le brevet européen: EP 0 336 841.

L'acide tert-butoxycarbonyl-3 diméthyl-2,2 [α-méthyl (méthoxy-4) benzyloxycarbonyl]-4 oxazolidinecarboxylique-5-(4S,5R) peut être préparé de la manière suivante :

A une solution de 2,1 g de tert-butoxycarbonyl-3 diméthyl-2,2 [α-méthyl (méthoxy-4) benzyloxycarbonyl]-4 oxazolidinecarboxylate-5-(4S,5R) de méthyle dans 55 cm3 d'éthanol, on ajoute à une température voisine de 25°C, une solution de 0,172 g d'hydrate d'hydroxyde de lithium dans 10 cm3 d'eau distillée. Le milieu réactionnel est agité pendant 2 heures à une température voisine de 20°C puis concentré à sec sous pression réduite (2,7 kPa) à une température voisine de 40°C. Le résidu d'évaporation est dissous dans 20 cm3 d'eau distillée et la solution obtenue est lavée par 2 fois 15 cm3 d'oxyde de diéthyle, acidifiée à un pH voisin de 2 par addition d'une solution aqueuse 0,5N d'acide chlorhydrique et extraite par 2 fois 100 cm3 d'acétate d'éthyle. Les phases organiques sont réunies, lavées par 2 fois 15 cm3 d'eau distillée, séchées sur sulfate de magnésium, filtrées puis concentrées à sec sous pression réduite (2,7 kPa) à une température voisine de 40°C. On obtient ainsi 1,9 g d'acide tert-butoxycarbonyl-3 diméthyl-2,2 [α-méthyl (méthoxy-4) benzyloxycarbonyl]-4 oxazolidinecarboxylique-5-(4S,5R) sous forme d'une meringue blanche.

Le tert-butoxycarbonyl-3 diméthyl-2,2 [α-méthyl (méthoxy-4) benzyloxycarbonyl]-4 oxazolidinecarboxylate-5-(4S,5R) de méthyle peut être préparé de la manière suivante :

A une solution de 2,76 g de tert-butoxycarbonyl-3 diméthyl-2,2 (carboxy-4 phényl)-4 oxazolidinecarboxylate-5-(4S,5R) de méthyle dans 180 cm3 de toluène anhydre on ajoute 3,01 g de N,N'-dicyclohexylcarbodiimide, 1,11 g de (méthoxy-4) phényl éthanol et 0,44 g de diméthylamino-4 pyridine. Le milieu réactionnel est ensuite maintenu sous agitation pendant 20 minutes à une température voisine de 20°C puis additionné d'un mélange de 40 cm3 de dichlorométhane et de 20 cm3 d'eau distillée. La phase aqueuse est séparée par décantation puis réextraite par 25 cm3 de dichlorométhane. Les phases organiques sont réunies, lavées par 2 fois 10 cm3 d'eau distillée, séchées sur du sulfate de magnésium, filtrées puis concentrées à sec sous pression réduite (2,7 kPa) à 40°C. On obtient ainsi 5,77 g d'une meringue blanche que l'on purifie par chromatographie sur 300 g de silice (0,063-0,2 mm) contenus dans une colonne de 3,2 cm de diamètre en éluant avec un mélange cyclohexane-acétate d'éthyle (90-10 en volumes) et en recueillant de fractions de 5 cm3. Les fractions ne contenant que le produit cherché sont réunies et concentrées à sec sous pression réduite (2,7 kPa) à une température voisine de 40°C. On obtient ainsi 2 g de tert-butoxycarbonyl-3 diméthyl-2,2 [α-méthyl (méthoxy-4) benzyloxycarbonyl]-4 oxazolidinecarboxylate-5-(4S,5R) de méthyle sous forme d'une huile jaune.

Le tert-butoxycarbonyl-3 diméthyl-2,2 (carboxy-4 phényl)-4 oxazolidinecarboxylate-5-(4S,5R) de méthyle peut être préparé de la manière suivante :

A une solution de 2,45 g de tert-butoxycarbonyl-3 diméthyl-2,2 (formyl-4) phényl-4 oxazolidinecarboxylate-5-(4S,5R) de méthyle dans 30 cm3 d'acide acétique on ajoute, à une température voisine de 20°C, 1,56 g d'hydrate de perborate de sodium. Le mélange réactionnel est ensuite agité à une température voisine de 45°C pendant 14 heures puis concentré à sec sous pression réduite (2,7 kPa) à 40°C. Le solide résiduel est dissous dans 100 cm3 d'acétate d'éthyle et la solution obtenue est lavée par 2 fois 10 cm3 d'eau distillée puis par 250 cm3 d'une solution aqueuse saturée d'hydrogénocarbonate de sodium. La phase aqueuse est lavée par 2 fois 10 cm3 d'éther diéthylique, acidifiée par 40 cm3 d'une solution aqueuse d'acide chlorhydrique 6N puis extraite par 2 fois 100 cm3 d'acétate d'éthyle. Les solutions organiques sont réunies, séchées sur du sulfate de magnésium, filtrées puis concentrées à sec sous pression réduite (2,7 kPa) à 40°C. On obtient ainsi 1,18 g de tert-butoxycarbonyl-3 diméthyl-2,2 (carboxy-4 phényl)-4 oxazolidine-carboxylate-5-(4S,5R) de méthyle sous forme d'une meringue blanche.

Le tert-butoxycarbonyl-3 diméthyl-2,2 (formyl-4 phényl)-4 oxazolidinecarboxylate-5-(4S,5R) de méthyle peut être préparé de la manière suivante :

A une solution de 7,55 g de tert-butoxycarbonyl-3 diméthyl-2,2 (vinyl-4 phényl)-4 oxazolidinecarboxylate-5-(4S,5R) de méthyle dans 190 cm3 de dichlorométhane on ajoute 1,7 cm3 de méthanol puis refroidit le milieu réactionnel à une température voisine de -78°C et fait passer pendant 4 heures, à une température voisine de -78°C et sous agitation, un léger courant d'ozone jusquà persistance de la coloration bleue. Le milieu réactionnel est ensuite agité à une température voisine de -78°C pendant 1 heure en faisant passer un léger courant d'air pour éliminer l'excès d'ozone puis on ajoute 6,2 cm3 de sulfure de diméthyle, réchauffe jusqu'à une température voisine de 20°C et maintient pendant 1 heure à cette température. Le mélange réactionnel est lavé par 2 fois 5 cm3 d'eau distillée, séché sur sulfate de magnésium, filtré puis concentré à sec sous pression réduite (2,7 kPa) à 40°C. On obtient 8,1 g d'une meringue blanche que l'on purifie par chromatographie sur 400 g de silice (0,063-0,2 mm) contenus dans une colonne de 5 cm de diamètre en éluant avec un mélange dichlorométhane-méthanol (99,5-0,5 en volumes) et en recueillant des fractions de 100 cm3. Les fractions ne contenant que le produit cherché sont réunies et concentrées à sec sous pression réduite (0,27 kPa) à 40°C. On obtient ainsi 4,96 g de tert-butoxycarbonyl-3 diméthyl-2,2 (formyl-4 phényl)-4 oxazolidinecarboxylate-5-(4S,5R) de méthyle sous forme d'une huile jaune.

Le tert-butoxycarbonyl-3 diméthyl-2,2 (vinyl-4 phényl)-4 oxazolidinecarboxylate-5-(4S,5R) de méthyle peut être préparé selon la méthode décrite dans l'exemple 6.

## Revendications

1. Procédé de préparation d'un taxoïde de formule générale : 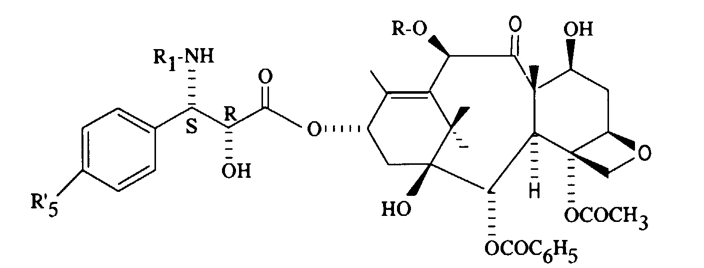 dans laquelle R représente un atome d'hydrogène ou un radical acétyle, R₁ représente un radical benzoyle ou un radical R₂-O-CO- dans lequel R₂ représente un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ), cycloalcoyle contenant 4 à 6 atomes de carbone, alcényle contenant 4 à 6 atomes de carbone, cyano, carboxy ou alcoyloxycarbonyle dont la partie alcoyle contient 1 à 4 atomes de carbone,
et R'₅ représente un radical alcényle contenant 2 à 8 atomes de carbone éventuellement substitué par un radical phényle, alcynyle contenant 2 atomes de carbone, phényle, formyle, alcanoyle, aroyle, hydroxyméthyl, carboxy ou alcoxycarbonyle, caractérisé en ce que :
a) on effectue l'iodation sélective d'un ester d'acide oxazolidinecarboxyliquede formule générale : 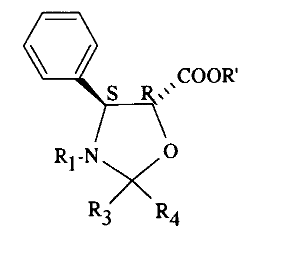 dans laquelle R₁ est défini comme précédemment, R₃ et R₄, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien R₃ représente un radical alcoxy contenant 1 à 4 atomes de carbone ou un radical trihalométhyle tel que trichlorométhyle ou un radical phényle substitué par un radical trihalométhyle tel que trichlorométhyle et R₄ représente un atome d'hydrogène, ou bien R₃ et R₄ forment ensemble avec l'atome de carbone auquel ils sont liés un cycle ayant 4 à 7 chaînons, et R' représente un radical alcoyle éventuellement substitué par un radical phényle, pour obtenir un ester de formule générale : 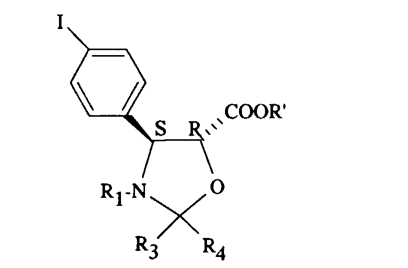 dans laquelle R₁, R₃, R₄ et R' sont définis comme ci-dessus,
b) on substitue l'atome d'iode de l'ester obtenu selon les méthodes connues de substitution pour obtenir un ester de formule générale : 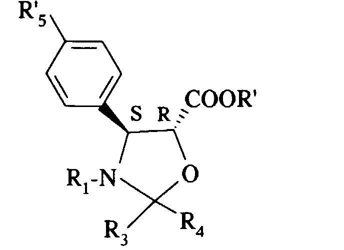 dans laquelle R₁, R₃, R₄, R' et R'₅ sont définis comme précédemment
c) on saponifie l'ester ainsi obtenu en acide oxazolidinecarboxylique,
d) on estérifie la baccatine III ou la désacétyl-10 baccatine III protégée au moyen de l'acide ainsi obtenu en opérant selon les méthodes connues,
e) on remplace les groupements protecteurs par des atomes d'hydrogène en passant éventuellement par un produit partiellement déprotégé dont on acyle la fonction amine avant de remplacer les groupements protecteurs restants par des atomes d'hydrogène.

2. Procédé de préparation d'un taxoïde de formule générale : 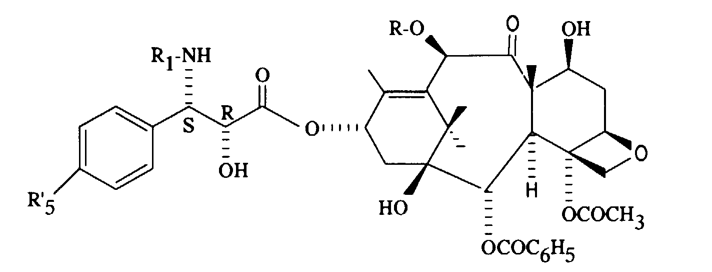 dans laquelle R représente un atome d'hydrogène ou un radical acétyle, R₁ représente un radical benzoyle ou un radical R₂-O-CO- dans lequel R₂ représente un radical alcoyle droit ou ramifié contenant 1 à 8 atomes de carbone, alcényle contenant 3 à 6 atomes de carbone, cycloalcoyle contenant 3 à 6 atomes de carbone, cycloalcényle contenant 4 à 6 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ), cycloalcoyle contenant 4 à 6 atomes de carbone, alcényle contenant 4 à 6 atomes de carbone, cyano, carboxy ou alcoyloxycarbonyle dont la partie alcoyle contient 1 à 4 atomes de carbone, R'₅ représente un radical alcényle contenant 2 à 8 atomes de carbone éventuellement substitué par un radical phényle, alcynyle contenant 2 atomes de carbone, phényle, formyle, alcanoyle, aroyle, hydroxyméthyl, carboxy ou alcoxycarbonyle, caractérisé en ce que :
a) on effectue une iodation sélective d'un ester d'acide oxazolidinecarboxylique de formule générale : 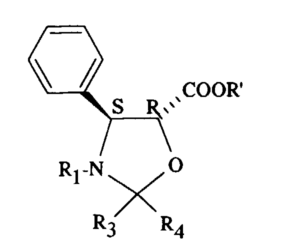 dans laquelle R' représente un radical alcoyle éventuellement substitué par un radical phényle et R₁, R₃ et R₄ sont définis comme dans la revendication 1 pour obtenir un ester de formule générale : 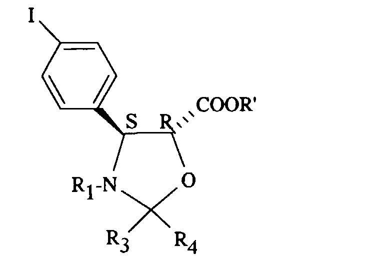 dans laquelle R' représente un radical alcoyle éventuellement substitué par un radical phényle et R₁, R₃ et R₄ sont définis comme dans la revendication 1,
b) on saponifie l'ester obtenu en acide correspondant,
c) on estérifie la baccatine III ou la désacétyl-10 baccatine III protégée au moyen de l'acide obtenu selon les méthodes connues,
d) on remplace les groupements protecteurs par des atomes d'hydrogène en passant éventuellement par un produit partiellement déprotégé dont on acyle la fonction amine avant de remplacer les groupements protecteurs restants par des atomes d'hydrogène, puis
e) on substitue l'atome d'iode par un substituant défini par R'₅

3. Procédé selon l'une des revendications 1 ou 2 caractérisé en ce que l'iodation sélective est effectuée au moyen d'iode en présence de bis-(trifluoroacétoxy) iodobenzène.

4. Procédé selon l'une des revendications 1 ou 2 caractérisé en ce que l'on opère dans un solvant organique.

5. Procédé selon la revendication 3 caractérisé en ce que le solvant est un hydrocarbure aliphatique halogéné.

6. Procédé selon l'une des revendications 1, 2, 3 ou 4 caractérisé en ce que l'on opère à une température comprise entre 0 et 50°C.

7. Procédé selon l'une des revendications 1 ou 2 caractérisé en ce que l'iodation est effectuée au moyen d'iode en présence de nitrate d'ammonium cérique en opérant dans l'acide acétique ou le méthanol.

8. Procédé selon l'une des revendications 1 ou 2 caractérisé en ce que l'iodation est effectuée au moyen d'iode en présence de trifluoroacétate d'argent.

9. Procédé selon l'une des revendications 1 ou 2 caractérisé en ce que l'iodation est effectuée au moyen de N-iodosuccinimide en présence d'hydroxy tosyloxy iodobenzène.

10. Procédé selon l'une des revendications 1 ou 2 caractérisé en ce que l'iodation est effectuée au moyen de dichloroiodure de benzyltriméthylammonium en présence de chlorure de zinc.

## Patentansprüche

1. Verfahren zur Herstellung eines Taxoids der allgemeinen Formel: 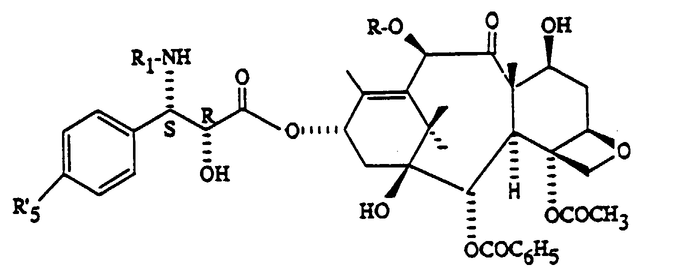 worin
R ein Wasserstoffatom oder einen Acetylrest darstellt,
R₁ einen Benzoylrest oder einen Rest R₂-O-CO- bedeutet, worin R₂ einen geraden oder verzweigten Alkylrest mit 1 bis 8 Kohlenstoffatomen, Alkenyl mit 3 bis 6 Kohlenstoffatomen, Cycloalkyl mit 3 bis 6 Kohlenstoffatomen, Cycloalkenyl mit 4 bis 6 Kohlenstoffatomen bedeutet, wobei diese Reste gegebenenfalls durch einen oder mehrere Substituenten substituiert sind, ausgewählt unter den Halogenatomen und den Hydroxyresten, Alkyloxy mit 1 bis 4 Kohlenstoffatomen, Dialkylamino mit 1 bis 4 Kohlenstoffatomen in jedem Alkylteil, Piperidino, Morpholino, 1-Piperazinyl (gegebenenfalls substituiert in 4-Stellung durch einen Alkylrest mit 1 bis 4 Kohlenstoffatomen), Cycloalkyl mit 4 bis 6 Kohlenstoffatomen, Alkenyl mit 4 bis 6 Kohlenstoffatomen, Cyano, Carboxy oder Alkyloxycarbonyl, dessen Alkylteil 1 bis 4 Kohlenstoffatome enthält, und
R₅' einen Alkenylrest mit 2 bis 8 Kohlenstoffatomen, gegebenenfalls substituiert durch einen Rest Phenyl, Alkinyl, enthaltend 2 Kohlenstoffatome, Phenyl, Formyl, Alkanoyl, Aroyl, Hydroxymethyl, Carboxy oder Alkoxycarbonyl bedeutet,
dadurch gekennzeichnet, daß man:
a) die selektive Jodierung eines Oxazolidincarbonsäureesters der allgemeinen Formel: 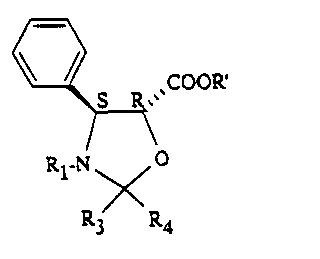 bewirkt, worin
R₁ wie vorstehend definiert ist,
R₃ und R₄, die identisch oder verschieden sind, ein Wasserstoffatom oder einen Alkylrest mit 1 bis 4 Kohlenstoffatomen darstellt, oder einen Aralkylrest, dessen Alkylteil 1 bis 4 Kohlenstoffatome enthält, und der Arylteil vorzugsweise einen Phenylrest darstellt, gegebenenfalls substituiert durch einen oder mehrere Alkoxyreste mit 1 bis 4 Kohlenstoffatomen, oder einen Arylrest bedeutet, vorzugsweise einen Phenylrest, gegebenenfalls substituiert durch einen oder mehrere Alkoxyreste mit 1 bis 4 Kohlenstoffatomen, oder auch R₃ einen Alkoxyrest mit 1 bis 4 Kohlenstoffatomen, oder einen Trihalomethylrest wie Trichloromethyl oder einen Phenylrest, substituiert durch einen Trihalomethylrest wie Trichloromethyl, bedeutet, und R₄ bedeutet ein Wasserstoffatom oder auch R₃ und R₄ bilden zusammen mit dem Kohlenstoffatom, an das sie gebunden sind, einen Ring mit 4 bis 7 Kettengliedern, und R' bedeutet einen Alkylrest, gegebenenfalls substituiert durch einen Phenylrest, um einen Ester der allgemeinen Formel: 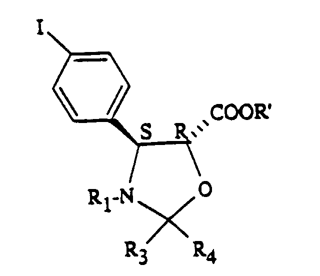 zu erhalten, worin R₁, R₃, R₄ und R' wie vorstehend definiert sind,
b) das Jodatom das erhaltenen Esters nach bekannten Substitutionsmethoden substituiert, um einen Ester der allgemeinen Formel: 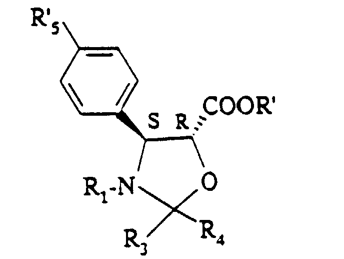 zu erhalten, worin R₁, R₃, R₄, R' und R'₅ wie vorstehend definiert sind;
c) den so erhaltenen Ester zur Oxazolidincarbonsäure verseift,
d) das Baccatin III oder das geschützte 10-Desacetyl-baccatin III mittels der so erhaltenen Säure verestert, wobei gemäß bekannten Methoden gearbeitet wird,
e) die Schutzgruppen durch Wasserstoffatome ersetzt, indem gegebenenfalls über ein teilweise von Schutzgruppen befreites Produkt gegangen wird, von dem man die Aminfunktion acyliert, bevor die restlichen Schutzgruppen durch Wasserstoffatome ersetzt werden.

2. Verfahren zur Herstellung eines Taxoids der allgemeinen Formel: 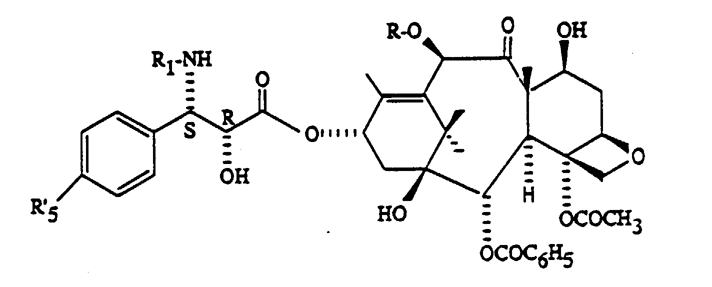 worin
R ein Wasserstoffatom oder einen Acetylrest darstellt,
R₁ einen Benzoylrest oder einen Rest R₂-O-CO- bedeutet, worin R₂ einen geraden oder verzweigten Alkylrest mit 1 bis 8 Kohlenstoffatomen, Alkenyl mit 3 bis 6 Kohlenstoffatomen, Cycloalkyl mit 3 bis 6 Kohlenstoffatomen, Cycloalkenyl mit 4 bis 6 Kohlenstoffatomen bedeutet, wobei diese Reste gegebenenfalls durch einen oder mehrere Substituenten substituiert sind, ausgewählt unter den Halogenatomen und den Hydroxyresten, Alkyloxy mit 1 bis 4 Kohlenstoffatomen, Dialkylamino mit 1 bis 4 Kohlenstoffatomen in jedem Alkylteil, Piperidino, Morpholino, 1-Piperazinyl (gegebenenfalls substituiert in 4-Stellung durch einen Alkylrest mit 1 bis 4 Kohlenstoffatomen), Cycloalkyl mit 4 bis 6 Kohlenstoffatomen, Alkenyl mit 4 bis 6 Kohlenstoffatomen, Cyano, Carboxy oder Alkyloxycarbonyl, dessen Alkylteil 1 bis 4 Kohlenstoffatome enthält,
R'₅ einen Alkenylrest mit 2 bis 8 Kohlenstoffatomen, gegebenenfalls substituiert durch einen Rest Phenyl, Alkinyl mit 2 Kohlenstoffatomen, Phenyl, Formyl, Alkanoyl, Aroyl, Hydroxymethyl, Carboxy oder Alkoxycarbonyl bedeutet,
dadurch gekennzeichnet, daß man:
a) die selektive Jodierung eines Oxazolidincarbonsäureesters der allgemeinen Formel: 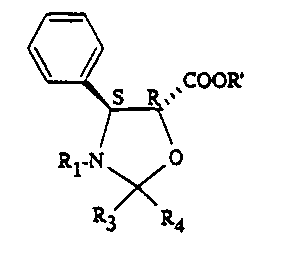 bewirkt, worin
R' einen gegebenenfalls durch einen Phenylrest substituierten Alkylrest darstellt, und R₁, R₃ und R₄ wie in Anspruch 1 definiert sind, um einen Ester der allgemeinen Formel: 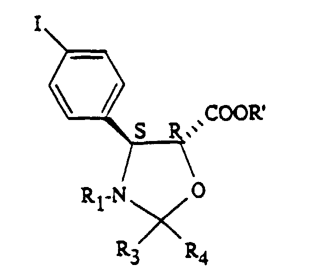 zu erhalten, worin R' einen gegebenenfalls durch einen Phenylrest substituierten Alkylrest bedeutet, und R₁, R₃ und R₄ wie in Anspruch 1 definiert sind,
b) den erhaltenen Ester zur entsprechenden Säure verseift,
c) das Baccatin III oder das geschützte 10-Desacetylbaccatin III mittels der erhaltenen Säure nach bekannten Methoden verestert,
d) die Schutzgruppen durch Wasserstoffatome ersetzt, wobei gegebenenfalls über ein Produkt gegangen wird, das teilweise von Schutzgruppen befreit ist, von dem man die Aminfunktion acyliert, bevor die restlichen Schutzgruppen durch Wasserstoffatome ersetzt werden,
dann
e) das Jodatom durch einen durch R'₅ definierten Substituenten substituiert.

3. Verfahren gemäß einem der Ansprüche 1 oder 2, dadurch gekennzeichnet, daß die selektive Jodierung mittels Jod in Gegenwart von Bis-(trifluoroacetoxy)-jodobenzol bewirkt wird.

4. Verfahren gemäß einem der Ansprüche 1 oder 2, dadurch gekennzeichnet, daß man in einem organischen Lösungsmittel arbeitet.

5. Verfahren gemäß Anspruch 3, dadurch gekennzeichnet, daß das Lösungsmittel ein halogenierter aliphatischen Kohlenwasserstoff ist.

6. Verfahren gemäß einem der Ansprüche 1, 2, 3 oder 4, dadurch gekennzeichnet, daß man bei einer Temperatur in dem Bereich zwischen 0 °C und 50 °C arbeitet.

7. Verfahren gemäß einem der Ansprüche 1 oder 2, dadurch gekennzeichnet, daß die Jodierung mittels Jod in Gegenwart von Cerammoniumnitrat bewirkt wird, wobei in Essigsäure oder Methanol gearbeitet wird.

8. Verfahren gemäß einem der Ansprüche 1 oder 2, dadurch gekennzeichnet, daß die Jodierung mittels Jod in Gegenwart von Silbertrifluoroacetat bewirkt wird.

9. Verfahren gemäß einem der Ansprüche 1 oder 2, dadurch gekennzeichnet, daß die Jodierung mittels N-Jodosuccinimid in Gegenwart von Hydroxytosyloxyjodobenzol bewirkt wird.

10. Verfahren gemäß einem der Ansprüche 1 oder 2, dadurch gekennzeichnet, daß die Jodierung mittels Benzyltrimethylammoniumdichlorojodid in Gegenwart von Zinkchlorid bewirkt wird.

## Claims

1. Process for the preparation of a taxoid of general formula: 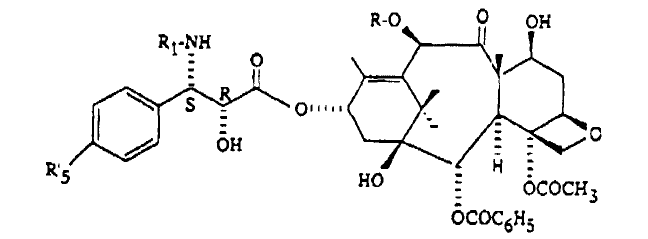 in which R represents a hydrogen atom or an acetyl radical, R₁ represents a benzoyl radical or a radical R₂-O-CO- in which R₂ represents a straight or branched alkyl radical containing 1 to 8 carbon atoms, an alkenyl radical containing 3 to 6 carbon atoms, a cycloalkyl radical containing 3 to 6 carbon atoms or a cycloalkenyl radical containing 4 to 6 carbon atoms, these radicals being optionally substituted with one or more substituents chosen from halogen atoms and hydroxyl radicals, alkyloxy radicals containing 1 to 4 carbon atoms, dialkylamino radicals in which each alkyl portion contains 1 to 4 carbon atoms, piperidino radicals, morpholino radicals, 1-piperazinyl radicals (optionally substituted at position 4 with an alkyl radical containing 1 to 4 carbon atoms) cycloalkyl radicals containing 4 to 6 carbon atoms, alkenyl radicals containing 4 to 6 carbon atoms, cyano radicals, carboxyl radicals or alkyloxycarbonyl radicals in which the alkyl portion contains 1 to 4 carbon atoms,
and R'₅ represents an alkenyl radical containing 2 to 8 carbon atoms optionally substituted with a phenyl radical, an alkynyl radical containing 2 carbon atoms or a phenyl, formyl, alkanoyl, aroyl, hydroxymethyl, carboxyl or alkoxycarbonyl radical, characterized in that:
a) a selective iodination is carried out on an oxazolidinecarboxylic acid ester of general formula: 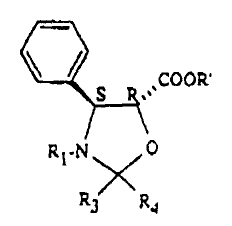 in which R₁ is defined as above and R₃ and R₄, which may be identical or different, represent a hydrogen atom or an alkyl radical containing 1 to 4 carbon atoms or an aralkyl radical in which the alkyl portion contains 1 to 4 carbon atoms and the aryl portion preferably represents a phenyl radical optionally substituted with one or more alkoxy radicals containing 1 to 4 carbon atoms, or an aryl radical preferably representing a phenyl radical optionally substituted with one or more alkoxy radicals containing 1 to 4 carbon atoms, or alternatively R₃ represents an alkoxy radical containing 1 to 4 carbon atoms or a trihalomethyl radical such as trichloromethyl or a phenyl radical substituted with a trihalomethyl radical such as trichloromethyl and R₄ represents a hydrogen atom, or alternatively R₃ and R₄ form, together with the carbon atom to which they are attached, a 4- to 7-membered ring, and R' represents an alkyl radical optionally substituted with a phenyl radical to obtain an ester of general formula : 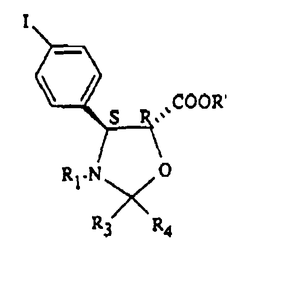 in which R₁, R₃, R₄ and R' are defined as above,
b) the iodine atom of the ester obtained is substituted according to known substitution methods in order to obtain an ester of general formula: 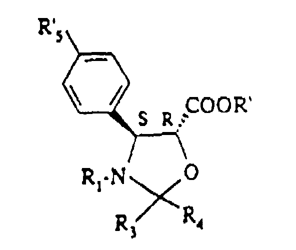 in which R₁, R₃, R₄, R' and R'₅ are defined as above,
c) the ester thus obtained is saponified to oxazolidinecarboxylic acid,
d) the protected baccatin III or 10-deacetylbaccatin III is esterified with the acid thus obtained, working according to known methods,
e) the protecting groups are replaced by hydrogen atoms, optionally passing via a partially deprotected product whose amine function is acylated before replacing the remaining protecting groups by hydrogen atoms.

2. Process for the preparation of a taxoid of general formula: 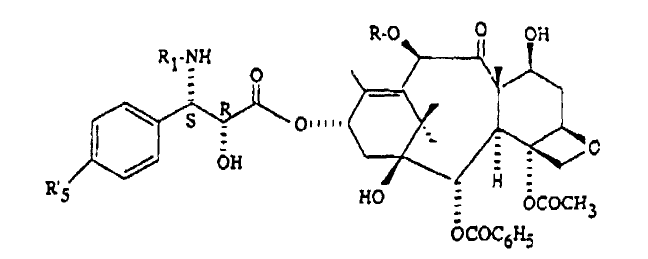 in which R represents a hydrogen atom or an acetyl radical, R₁ represents a benzoyl radical or a radical R₂-O-CO- in which R₂ represents a straight or branched alkyl radical containing 1 to 8 carbon atoms, an alkenyl radical containing 3 to 6 carbon atoms, a cycloalkyl radical containing 3 to 6 carbon atoms or a cycloalkenyl radical containing 4 to 6 carbon atoms, these radicals being optionally substituted with one or more substituents chosen from halogen atoms and hydroxyl radicals, alkyloxy radicals containing 1 to 4 carbon atoms, dialkylamino radicals in which each alkyl portion contains 1 to 4 carbon atoms, piperidino radicals, morpholino radicals, 1-piperazinyl radicals (optionally substituted at position 4 with an alkyl radical containing 1 to 4 carbon atoms) cycloalkyl radicals containing 4 to 6 carbon atoms, alkenyl radicals containing 4 to 6 carbon atoms, cyano radicals, carboxyl radicals or alkyloxycarbonyl radicals in which the alkyl portion contains 1 to 4 carbon atoms,
and R'₅ represents an alkenyl radical containing 2 to 8 carbon atoms optionally substituted with a phenyl radical, an alkynyl radical containing 2 carbon atoms or a phenyl, formyl, alkanoyl, aroyl, hydroxymethyl, carboxyl or alkoxycarbonyl radical, characterized in that:
a) a selective iodination is carried out on an oxazolidinecarboxylic acid ester of general formula: 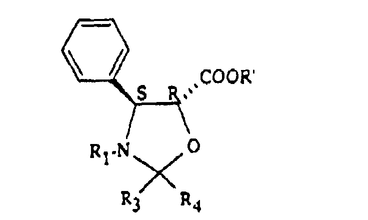 in which R' represents an alkyl radical optionally substituted with a phenyl radical and R₁, R₃ and R₄ are defined as in claim 1, to obtain an ester of general formula: 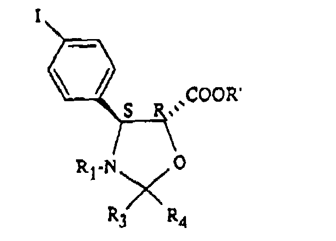 in which R' represents an alkyl radical optionally substituted with a phenyl radical and R₁, R₃ and R₄ are defined as in claim 1,
b) the ester obtained is saponified to the corresponding acid,
c) the protected baccatin III or 10-deacetylbaccatin III is esterified with the acid obtained, according to known methods,
d) the protecting groups are replaced by hydrogen atoms, optionally passing via a partially deprotected product whose amine function is acylated before replacing the remaining protecting groups by hydrogen atoms, then
e) the iodine atom is substituted with a substituent defined by R'₅.

3. Process according to either of claims 1 and 2, characterized in that the selective iodination is carried out using iodine in the presence of bis (trifluoroacetoxy) iodobenzene.

4. Process according to either of claims 1 and 2, characterized in that the process is performed in an organic solvent.

5. Process according to claim 4, characterized in that the solvent is a halogenated aliphatic hydrocarbon.

6. Process according to one of claims 1, 2, 3 and 4, characterized in that the process is performed at a temperature between 0 and 50°C.

7. Process according to either of claims 1 and 2, characterized in that the iodination is carried out using iodine in the presence of ammonium cerium nitrate and by working in acetic acid or methanol.

8. Process according to either of claims 1 and 2, characterized in that the iodination is carried out using iodine in the presence of silver trifluoroacetate.

9. Process according to either of claims 1 and 2, characterized in that the iodination is carried out using N-iodosuccinimide in the presence of hydroxy (tosyloxy)iodobenzene.

10. Process according to one of claims 1 and 2, characterized in that the iodination is carried out using benzyltrimethylammonium dichloroiodide in the presence of zinc chloride.
